# EUROPEAN PATENT SPECIFICATION

(11) **EP 2 231 904 B1**
(45) Date of publication and mention of the grant of the patent: **13.01.2016**
(21) Application number: 08867683.8
(22) Date of filing: 21.11.2008
(51) Int. Cl.: C40B 40/02

(54) **DESIGN AND GENERATION OF HUMAN DE NOVO PIX PHAGE DISPLAY LIBRARIES VIA FUSION TO PIX OR PVII, VECTORS, ANTIBODIES AND METHODS**
DESIGN UND ERSTELLUNG HUMANER DE NOVO PIX-PHAGEN-DISPLAY-BIBLIOTHEKEN MITTELS FUSION AN PIX ODER PVII, VEKTOREN, ANTIKÖRPER UND VERFAHREN
CONCEPTION ET GÉNÉRATION DE BANQUES D'EXPOSITION SUR PHAGE HUMAIN PIX DE NOVO AU MOYEN D'UNE FUSION VERS PIX OU PVII, VECTEUR, ANTICORPS ET PROCÉDÉS

(30) Priority: 19.12.2007 US 14786 P
(43) Date of publication of application: 29.09.2010
(73) Proprietor: Janssen Biotech, Inc., Horsham, PA 19044 (US)
(72) Inventor: TSUI, Ping, Berwyn, PA 19312 (US); SHI, Lei, Malvern, PA 19355 (US); LU, Jin, Malvern, PA 19355 (US); WHEELER, John, Malvern, PA 19355 (US); WHITAKER, Brian, Malvern, PA 19355 (US); BOROZDINA-BIRCH, Lionella, Malvern, PA 19344 (US); ALMAGRO, Juan, Malvern, PA 19355 (US); AMEGADZIE, Bernard, Malvern, PA 19355 (US); TORNETTA, Mark, Malvern, PA 19355 (US); REDDY, Ramachandra, Malvern, PA 19355 (US); KNIGHT, David, Malvern, PA 19355 (US); LUO, Jinquan, Malvern, PA 19355 (US); SWEET, Raymond, Malvern, PA 19355 (US)
(74) Representative: Goodfellow, Hugh Robin
(86) International application number: PCT/US2008/084255
(87) International publication number: WO 2009/085462

(56) References cited:
- US-A- 5 658 727
- US-A- 5 658 727
- US-A1- 2003 186 322
- US-A1- 2003 186 322
- GAO C ET AL: "A method for the generation of combinatorial antibody libraries using pIX phage display", PROCEEDINGS OF THE NATIONAL ACADEMY OF SCIENCES OF THE UNITED STATES (PNAS), NATIONAL ACADEMY OF SCIENCE, US, vol. 99, no. 20, 1 October 2002 (2002-10-01), pages 12612-12616, XP002583225, ISSN: 0027-8424, DOI: DOI:10.1073/PNAS.192467999 [retrieved on 2002-09-18]
- KUNKEL T A ET AL: "EFFICIENT SITE-DIRECTED MUTAGENESIS USING URACIL-CONTAINING DNA", METHODS IN ENZYMOLOGY; [METHODS IN ENZYMOLOGY], ACADEMIC PRESS INC, SAN DIEGO, CA, US, vol. 204, 1 January 1991 (1991-01-01), pages 125-139, XP009073170, ISSN: 0076-6879, DOI: DOI:10.1016/0076-6879(91)04008-C
- GAO CHANGSHOU ET AL: "Making artificial antibodies: A format for phage display of combinatorial heterodimeric arrays", PROCEEDINGS OF THE NATIONAL ACADEMY OF SCIENCES OF THE UNITED STATES (PNAS), NATIONAL ACADEMY OF SCIENCE, US, vol. 96, no. 11, 25 May 1999 (1999-05-25), pages 6025-6030, XP002245749, ISSN: 0027-8424, DOI: DOI:10.1073/PNAS.96.11.6025
- GAO CHANGSHOU ET AL: "A cell-penetrating peptide from a novel pVII-pIX phage-displayed random peptide library", BIOORGANIC & MEDICINAL CHEMISTRY, PERGAMON, GB, vol. 10, no. 12, 1 December 2002 (2002-12-01), pages 4057-4065, XP002404392, ISSN: 0968-0896, DOI: DOI:10.1016/S0968-0896(02)00340-1
- KWASNIKOWSKI ET AL: "Multivalent display system on filamentous bacteriophage pVII minor coat protein", JOURNAL OF IMMUNOLOGICAL METHODS, ELSEVIER SCIENCE PUBLISHERS B.V.,AMSTERDAM, NL, vol. 307, no. 1-2, 20 December 2005 (2005-12-20), pages 135-143, XP005221142, ISSN: 0022-1759, DOI: DOI:10.1016/J.JIM.2005.10.002
- DE HAARD H J ET AL: "A large non-immunized human Fab fragment phage library that permits rapid isolation and kinetic analysis of high affinity antibodies", JOURNAL OF BIOLOGICAL CHEMISTRY, AMERICAN SOCIETY FOR BIOCHEMISTRY AND MOLECULAR BIOLOGY, US, vol. 274, no. 26, 25 June 1999 (1999-06-25), pages 18218-18230, XP002128301, ISSN: 0021-9258, DOI: 10.1074/JBC.274.26.18218
- OH ET AL: "Enhancing phage display of antibody fragments using gIII-amber suppression", GENE, ELSEVIER, AMSTERDAM, NL, vol. 386, no. 1-2, 19 December 2006 (2006-12-19), pages 81-89, XP005808302, ISSN: 0378-1119, DOI: 10.1016/J.GENE.2006.08.009

## Description

### FIELD OF THE INVENTION

The invention relates to a compositions and methods for generating and using pIX phage display libraries for producing antibodies or antibody fragments.

### BACKGROUND OF THE INVENTION

Filamentous phage display using pIII and pVIII as fusion partners in phage or phagemid systems have been used as a technology for protein engineering, notably for *de novo* antibody isolation and affinity maturation. Human-like fab sequences can be generated from known antibody sequences as templates and random or mutangenized complementarity determining region (CDR) or antigen binding regions, such as heavy chain CDR3 (H3), can be generated and isolated from phage libraries displaying variations of antibody fragment sequences via panning against an antigen or other protein target of interest without immunization. Previously used human de novo antibody libraries have been created synthetically or by molecular cloning of IgG genes from naive source(s). In a synthetic library, antibody DNA sequences including variable heavy chain and light chain framework and the CDR regions are designed and synthesized based on 1) a defined IgG gene, 2) a specific Ig germline gene, and 3) consensus sequences from families of the Ig germline genes and 4) PCR derived IgG fragments from a natural sources. In addition to the synthetic antibody library, libraries can also be created by combinatorial cloning of IgG DNA derived from human tissues, e.g., bone marrows and peripheral blood cells. Such libraries have been used for providing fab antibody fragments and for running successive rounds of panning and maturation or modification to attempt to find fab antibody fragments that have desired properties such as high affinity or inhibitory biological activity of a selected target protein.

Human like fab antibodies to several target proteins have been isolated from phage display pIII or pVIII antibody libraries. Although successful at isolating fab antibodies to specific targets, such phage display library approaches suffer from the problems of having to repeat the process of library generation, panning and maturation several times to isolate fab antibodies having the desired characteristics. Such phage libraries also suffer from the problem that they do not fully encompass or mimic the range of human immune diversity present in humans, including the contribution of VH/VL paring, topology features of different germline families related to antigen recognition, the position and extent of amino acid variation, and the relative abundance of antibodies derived from different human germline genes. Deviation of synthetic antibodies from the natural repertoire may increase the risk of unfavorable biochemical properties and of immunogenicity if used as therapeutics in man.

There is a need for synthetic antibody libraries and methods that simultaneously deliver the critical elements of human therapeutic antibodies of high affinity and activity, high productivity, good solution properties, and a propensity of low immune response when administered in man. There is a further need to increase the efficiency of antibody isolation from synthetic libraries, relative to current methods, to reduce the resource costs of antibody discovery and accelerate delivery of antibodies for biological evaluation. The libraries and methods of this invention meet these needs by coupling comprehensive design, assembly technologies, and phage pIX Fab display.

US 2003/186322 describes the display of exogenous polypeptides on filamentous phage using a fusion between the exogenous polypeptide and phage pVII or pIX proteins. Gao et al. (PNAS, 2002, vol. 99(20) 12612-12616) constructed a large, human single-chain Fv (scFv) library of 4.5 x 10(9) members displayed on pIX. US 5 658 727 discloses filamentous phage comprising a matrix of cpVIII proteins. Kunkel et al. (Methods in Enzymology, 1991, vol. 204, 125-139) discloses efficient site-directed mutagenesis using uracil-containing DNA.

### SUMMARY OF THE INVENTION

The invention provides an engineered recombinant nucleic acid phage vector for expressing phage display pIX fusion antibody fab fragment amino acid sequences that bind to selected biologically active ligands, comprising: (i) a) a recombinant phage leader coding nucleic acid sequence; operably linked to: b) a recombinant tag, promoter, or selection coding nucleic acid sequence; operably linked to: c) a variable heavy chain germline encoding nucleic acid sequence that selectively binds to said biologically active ligand; operably linked to: d) a portion of a constant heavy chain germline encoding nucleic acid sequence that selectively binds to said biologically active ligand; operably linked to: e) a peptide linker encoding nucleic acid sequence; operably linked to: f) a recombinant pIX encoding nucleic acid sequence; and (ii) a) a variable light chain germline encoding nucleic acid sequence that selectively binds to said biologically active ligand; operably linked to: b) a portion of a constant light chain germline encoding nucleic acid sequence that selectively binds to said biologically active ligand.

The invention also provides a phage library of bacterial host cells comprising a plurality of engineered nucleic acid phage vectors according to the invention.

The invention also provides a method for screening a phage fab antibody fragment library for fab antibody fragments having a desired biological activity, comprising (a) expressing fab antibody fragments from a phage library according to the invention, and (b) selecting bacterial cells expressing said fab antibody fragments having said desired biological activity.

### SUMMARY OF THE DISCLOSURE

In contrast to the teaching of the prior art, it has now been discovered that pVII and pIX can successfully be used for generating high affinity fab libraries, e.g., using mutagenesis or other diversity producing techniques, optionally with in line maturation, to provide an efficient and fast platform for fab and antibody fragment generation and selection of therapeutic antibodies. According to the present disclosure, antibody variable or fab regions fused to pVII and pIX engage in a dynamic interaction on the phage surface to display a functional antibody fragment, a representative heterodimeric motif. The display on phage of antibody heavy and light chain variable regions is therefore a suitable and preferred method for display and assay of diverse libraries of combinatorial heterodimeric arrays in which members can function as dimeric artificial antibody species and allow for selection of novel or desired biological activities.

Disclosed herein are various improved and new pIX and pVII phage display de novo library generation methods and components, such as but not limited to, one or more of (i) designed and displayed antibody Fab de novo libraries fused to the pIX or pVII phage proteins; (ii) the use of a phage surface protein different from the widely used pIII and pVIII of M13 phage; (iii) the use of a small array of germline VH and VL genes representing the sequence and structure of human repertoire; (iv) use of such phage components as the library scaffold to provide improved designed, combinatorial diversities in the complimentarity regions of the Vh and VI regions; (v) antibody selection processes that allow systematic examination of the effect of the designed sequences and structural topologies for antigen recognition; (vi) a streamlined affinity maturation and in line maturation process as a part of the library selection. Such a new system of library design, selection, optimization and maturation of individual or groups of libraries provide a reproducible and reliable system for successful antibody de novo discovery and also facilitate understanding the structure function relation of antibody to antigen interaction.

The human Fab *de novo* library described above is distinct from current antibody library state-of-the-art by its display via the pIX gene of M13 phage. The use of representative human germline and structure sequences as the library scaffolds and the design of CDR sequences mimicking the natural amino acids distribution comprehensively cover the human immune repertoire, and the library antibodies have high sequence identity to the natural derived human antibodies. The comprehensive coverage of the human immune repertoire increases the chance of antibody *de novo* discovery comparing to the libraries built on a single germline or IgG gene scaffold reported in literature. The separately made sub-libraries, each carries a unique scaffold (VH/VL pair) and/or the H-CDR3 lengths, maximize the chance of identifying unique antibody and provide a mechanism to systematic examine antibody/antigen binding structure and function relationships. The integrated affinity maturation process will reduce time needed to discover diverse and high affinity antibodies.

Artificial antibodies are here defined as protein motifs of large diversity that use the functional strategy of the antibody molecule, but can be generated free of in vivo constraints, including (1) sequence homology and toxicity of target antigens; (2) biological impact of the generated antibody in the host or in hybridoma cultures used to recover the antibody; and (3) screening versus selection for desired activity. The antibody molecule is a biological device for the presentation of a combinatorial array of peptide elements in three-dimensional space. The essential feature is that while CDRs (complementarity determining regions) cooperate to form a binding site, their interaction is dynamic and functional with little structural association between the CDRs themselves. In this way, the full complement of amino acid residues are available for antigen recognition at a minimum energetic cost for binding. It is proposed that the ability to control the combinatorial design of not only sequence space, but also three-dimensional space, would recapitulate and ultimately transcend the natural design of the immune repertoire.

Thus the disclosure describes a combinatorial phage display format for construction of highly diverse heterodimeric polypeptide arrays. In particular, the disclosure describes a filamentous phage particle encapsulating a genome encoding a fusion polypeptide, wherein the fusion polypeptide comprises an exogenous polypeptide fused to the amino terminus of a filamentous phage pVII or pIX protein. Preferably, the phage particle comprises the expressed fusion protein on the surface of the phage particle.

In a preferred embodiment, the phage genome further encodes a second fusion polypeptide, wherein the second fusion polypeptide comprises a second exogenous polypeptide fused to the amino terminus of the pIX protein and the first exogenous polypeptide in the first fusion polypeptide is fused to the amino terminus of the pVII protein. In this embodiment, the first and second fusion polypeptides can associate to form a heterodimeric protein complex, such as an immunoglobulin Fv, a catalytic Fv, a receptor, a nucleic acid binding protein or an enzyme.

In a related embodiment, the disclosure describes a vector for expressing a fusion protein on the surface of a filamentous phage comprising a cassette for expressing the fusion protein. The cassette includes upstream and downstream translatable DNA sequences operatively linked via a sequence of nucleotides adapted for directional ligation of an insert DNA, i.e., a polylinker, where the upstream sequence encodes a prokaryotic secretion signal, the downstream sequence encodes a pVII or pIX filamentous phage protein. The translatable DNA sequences are operatively linked to a set of DNA expression signals for expression of the translatable DNA sequences as portions of the fusion polypeptide. In a preferred variation, the vector further comprises a second cassette for expressing a second fusion protein on the surface of the filamentous phage, wherein the second cassette has the structure of the first cassette with the proviso that the first fusion protein expression cassette encodes pVII protein and the second fusion protein expression cassette encodes pIX protein. The vector is used as a phage genome to express heterodimeric protein complexes on the surface of the phage particle in which the two exogenous polypeptides of the heterodimer are anchored on the phage particle by the fusion to the first and second phage proteins, pVII and pIX, respectively.

In another embodiment, the invention contemplates a library of phage particles according to the present invention, i.e., a combinatorial library, in which representative particles in the library each display a different fusion protein. Where the particle displays a heterodimeric protein complex, the library comprises a combinatorial library of heterodimers, such as antibodies in the form of a library of Fv molecules. Preferred libraries have a combinatorial diversity of at least 10³, 10⁴, 10⁵, 10⁶, 10⁷, 10⁸, 10⁹, 10¹⁰, 10¹¹, 10¹², 10¹³, or any range or value therein, different species of fusion protein.

A related embodiment describes a fusion protein comprising first and second polypeptides wherein the first polypeptide is an exogenous protein and the second polypeptide is a filamentous phage pVII or pIX protein, wherein the exogenous protein is fused to the amino terminus of the filamentous phage protein.

Still further, the disclosure contemplates a variety of methods for producing a combinatorial library of phage, including by cloning repertoires of genes encoding an exogenous polypeptide into a vector of the present invention, modifying the structure of the exogenous polypeptides in a library by mutagenesis, by random combination of populations of first and second fusion protein libraries, by target and affinity selection ("panning") to alter the diversity of a library, and the like.

The design of proteins with improved or novel functions is an important goal with a variety of medical, industrial, environmental, and basic research applications. Following the development of combinatorial antibody libraries, a powerful next step is the evolution toward artificial antibody constructs as well as other protein motifs in which dimeric species are native or might be functional.

The present disclosure addresses these challenges by providing a phage-display format for the construction of combinatorial heterodimeric polypeptide arrays in which pVII and pIX are utilized for the display of fusion proteins that form dimeric species. It is important to note that this is an entirely new methodology because one can independently display two protein motifs in close proximity to generate a library of functional interactions.

Inherent in the scope and power of the technology is the ability to display a variety of proteins that can engage in dimeric interactions. These include not only antibodies, but also some enzymes, hormones and hormone receptors, and DNA-binding proteins. The display technology described herein can be used for combinatorial alteration of antibody framework regions and to reorganize and miniaturize the antibody structure or to display DNA binding proteins, such as repressors, as a library of heterodimers for selection against particular DNA sequences of clinical and therapeutic importance.

Thus the present technology provides for the display and selection of mutant dimeric proteins and combinatorial libraries in which members consist of heterodimeric arrays. Using this technology, the native immunoglobulin structure, in a heterodimeric V.sub.H-V.sub.L Fv format shown herein, can be modified in different ways and screened for specificity and activity. For example, by combinatorial alteration of framework regions (FRs) or other manipulations to reorganize and miniaturize the antibody structure by processes coined "complementarity determining region (CDR) shuffling" and "twinibody" formation, antibody-like secondary structures will emerge that contain new paratopes or entirely different structural elements. Selection for binding and/or catalysis against the natural antigen and/or substrate as well as some related compounds will be used to screen the libraries of heterodimeric proteins.

Furthermore, sequence randomizations to form libraries and chain-shuffling protocols to form hybrid species can lead to subsets of novel proteins. For instance, the display and modification of arrays of zinc-finger domains in homodimeric or heterodimeric form produces structures that possess specific DNA interactions. In addition, entirely new constructs are possible via the insertion of a desired encoding fragment within a preformed scaffold such as an antibody chain. Possible insertions include an enzyme signature sequence or a repressor binding protein.

It should be understood that both the foregoing general description and the following detailed description are exemplary and explanatory only and are not restrictive of the invention as claimed.

### DESCRIPTION OF THE FIGURES

Figure 1. Scaffold VH and VL amino acid sequences.
Figure 2. Vector diagram of phage display and Fab expression vector: pCNTO-Fab-pIX-lacl.
Figure 3A-C: Graphical representation and image of Library scaffold Fab expression and display. 3A shows relative levels of Fab expression on the surface of M13 3B shows relative expression of Fabs in a Western blot using anti-Fab'(2) as a detection reagent. 3C shows approximate expression of Fab scaffolds in E.coli media supernatant by comparison to a Fab of known concentration. This includes the kappa gene scaffolds (solid bars) and lambda gene scaffolds (shaded bars).
Figure 4. Schematic of H-CDR3 design pattern.
Figure 5. Schematic of H-CDR3 oligonucleotide design.
Figure 6. Amino acid distribution in H-CDR3 positions for the H1-69 template. A. Distribution at 393 D positions (length 9-14). B. Distribution at 2163 N positions (length 9-14). C. Distribution at 973 Y positions in H-CDR3 (length 9-14); D. Distribution at all positions in H-CDR3 length 7 and 8 using NNS codon.
Figure 7. Summary diagram of Library architectures.
Figure 8. Schematic of palindrome assisted parental strand elimination.
Figure 9. Schematic of mega-primer targeting the entire region of a V region.
Figure 10. VH fragment from hit(s) is amplified by PCR and cloned into a VL-loop vector. The resulting plasmid is produced as ssDNA and serves as a template for megaprimer hybridization & digestion mutagenesis. The Lc library megaprimer is used to replicate the plasmid, thus incorporating the Lc library diversity in association with the isolated Hc sequence. This affinity maturation library is packaged as M13 phage and utilized for phage panning against the initial antigen to yield affinity-matured hits.

### DESCRIPTION OF THE INVENTION

The present disclosure provides various new phage display de novo library generation methods and components, such as but not limited to (i) designed and displayed antibody Fab de novo libraries fused to the pIX or other phage proteins; (ii) the use of a phage surface protein different from the widely used pIII and pVIII of M13 phage; (iii) the use of a small array of germline VH and VL genes representing the sequence and structure of human repertoire; (iv) use of such phage components as the library scaffold to provide improved designed, combinatorial diversities in the complimentarity regions of the Vh and VI regions; (v) antibody selection processes that allow systematical examination of the effect of the designed sequences and structural topologies for antigen recognition; (vi) a streamlined affinity maturation process as a part of the library selection. Such a new system of library design, selection, optimization and maturation of individual or groups of libraries provide a reproducible and reliable system for successful antibody de novo discovery and also facilitate understanding the structure function relation of antibody to antigen interaction.

The human Fab *de novo* library described above is distinct from current antibody library state-of-the-art by its displaying via pIX gene of M13 phage. The use of representative human germline and structure sequences as the library scaffolds and the design of CDR sequences mimicking the natural length and amino acid distribution comprehensively cover the human immune repertoire, and the library antibodies are highly homologue to the natural derived human antibodies. The comprehensive coverage of immune repertoire increases the chance of antibody *de novo* discovery comparing to the libraries built on a single germline or IgG gene scaffold reported in literature. The separately made sub-libraries, each carrying a unique scaffold (e.g., VH/VL pair and/or the H-CDR3 lengths), maximize the chance of identifying unique antibody and provide a mechanism to systematic examine antibody/antigen binding structure and function relationships. The integrated affinity maturation process will reduce time needed to discover diverse and high affinity antibodies.

### Definitions:

Antibody: The term antibody in its various grammatical forms is used herein to refer to immunoglobulin molecules and immunologically active portions of immunoglobulin molecules, i.e., molecules that contain an antibody combining site or paratope. Exemplary antibody molecules are intact immunoglobulin molecules, substantially intact immunoglobulin molecules and portions of an immunoglobulin molecule, including those portions known in the art as Fab, Fab', F(ab').sub.2 and Fv.

Antibody Combining Site: An antibody combining site is that structural portion of an antibody molecule comprised of a heavy and light chain-variable and hypervariable regions that specifically binds (immunoreacts with) an antigen. The term immunoreact in its various forms means specific binding between an antigenic determinant-containing molecule and a molecule containing an antibody combining site such as a whole antibody molecule or a portion thereof.

Fusion Polypeptide: A polypeptide comprised of at least two polypeptides and a linking sequence to operatively link the two polypeptides into one continuous polypeptide. The two polypeptides linked in a fusion polypeptide are typically derived from two independent sources, and therefore a fusion polypeptide comprises two linked polypeptides not normally found linked in nature.

Cistron: Sequence of nucleotides in a DNA molecule coding for an amino acid residue sequence and including upstream and downstream DNA expression control elements.

### ]Filamentous Phage

The present disclosure contemplates a filamentous phage comprising a matrix of proteins encapsulating a genome encoding a fusion protein (protein). The fusion protein comprises an exogenous polypeptide portion fused to the amino terminus of a filamentous phage pVII or pIX protein.

By "exogenous" is meant that the polypeptide fused to the phage protein is not normally associated with the phage pVII or pIX protein in wild-type varieties of filamentous phage, but rather are foreign to the normal phage protein.

A typical exogenous polypeptide is any polypeptide of interest, including an immunoglobulin heavy chain variable domain (V.sub.H), an immunoglobulin light chain variable domain (V.sub.L), natural or synthetic polypeptides, a single chain antibody (scFv), and the like.

In a preferred embodiment, a filamentous phage encapsulates a genome which encodes a first and second fusion protein, where the first fusion protein comprises a first exogenous polypeptide fused to pVII and the second fusion protein comprises a second exogenous polypeptide fused to pIX.

The filamentous phage will further contain the fusion protein(s) displayed on the surface of the phage particle, as described in the Examples. Thus, where there are first and second fusion proteins, the phage can display these proteins in a function manner such that the first and second exogenous polypeptides can interact as a heterodimer to form a functional two-chain protein complex on the phage surface.

Where an expressed heterodimeric protein has the capacity to bind ligand it is alternatively referred to herein as a ligand-binding heterodimeric receptor.

The heterodimeric receptor in a preferred embodiment is an epitope-binding complex. That is, a complex of first and second polypeptides capable of binding an epitope. Preferably, the first and second polypeptides are antibody heavy chain and light chain polypeptides. In particular, a preferred embodiment utilizes V.sub.H and V.sub.L to form an Fv complex. Other heterodimeric protein complexes include a catalytic Fv, a receptor, a nucleic acid binding protein, and enzyme and the like heterodimeric proteins.

In a fusion protein present on a phage disclosed herein, the "fusion" between the exogenous polypeptide and the filamentous phage pVII or pIX protein may comprise a typical amide linkage, or may comprise a linker polypeptide (i.e., a "linker") as described in the Examples. Any of a variety of linkers may be used which are typically a stretch of about 5 to 50 amino acids in length. Particularly preferred linkers provide a high degree of mobility to the fusion protein at the point of the linker.

Library design: prior synthetic libraries have incorporated some of the following, but none have included all in a comprehensive manner.

Germline V-region family diversity. The human variable repertoire for heavy and light chains consists of families of related sequences that are defined by sequence homology and length. In any such family, individual members primarily differ in sequence and length of the complementarity determining regions. These differences lead to known structural variation patterns (canonical structure) as well as germline amino acid variation at structurally similar positions that are predisposed to interact with antigen. Choosing only a single Vh and VI gene template for a library diminishes the scope of natural germline diversity that can be captured (cite Genentech libraries). Choosing all Vh and VI genes paralyzes the efficiency of library generation. The libraries of the invention efficiently capture the germline diversity by (1) identifying a small number of germline Vh, Vk, and V-lambda that represent the dominant canonical structure groups in rearranged human antibodies (IMGT, Kabat, NCBI) and (2) incorporating the natural human germline diversity of related family members in the VH gene encoded CDRs 1 and 2, or also in CDRs 1-3 of the Vk and V-lambda regions, by combinatorial oligonucleotide mutagenesis.

VH-CDR3 diversity. VH-CDR3 is created by the joining of the V, D and J segments and is accompanied by both end addition and exonucleolytic events. There are about 25 germline D region segments and this number coupled with the complex joining events and somatic mutation creates the most diverse region of antibodies. These events occur on a defined set of germline sequences and so are not random; but they are difficult to predict. However, the database of rearranged human antibody sequences now has reached sufficient size (about 5000 VH regions) to apply statistical evaluation of both length and amino acid distribution at each position in VH-CDR3. The libraries of the current invention recapitulate this natural human diversity by utilizing designed degenerate oligonucleotides to assemble this region.

Position and nature of somatic diversity. Somatic diversity is a hallmark of how human antibodies mature to high-affinity, selective binding entities. This generation and accumulation of somatic mutations is not random. The site and type of nucleotide mutation are biased by DNA sequence and mechanism but only mutations that provide binding and functional advantage are selected and stored, often along with neutral substitutions. While not amenable to prediction from mechanism, the database of rearranged human antibody sequences and structure-function analysis does identify positions and amino substitutions most frequently associated with recognition of antigen in CDR regions, including differentiation between protein, peptide and small molecule antigens. The libraries of the current invention recapitulate this natural human diversity by utilizing designed degenerate oligonucleotides to incorporate substitutions into VH CDR1 and 2, or also CDRs 1-3 of the Vk and V-lambda regions.

Germline gene usage. The human germline repertoire consists of about 30 V-kappa, 56 V-lambda, and 40 V-heavy chain functional genes. However, their representation in rearranged antibodies is strongly biased and this is reflected in the frequency of pairing of different light heavy chain V-regions (de Wildt et al., J Mol Bio 285: 895-901 (1999)). The libraries of the invention capture this bias by selecting a dominant germline V-region for each of the diversity classes noted in (a) above.

Expression, biochemical, and biophysical properties. Preferred human antibodies have desired biological and binding activities, but also are efficiently produced from a variety of hosts, are stable, and have good solution properties. High-frequency germline gene usage (1d) also indicates good expression in mammalian systems. In addition, antibodies recovered from libraries by bacterial phage display methods of selection or screening should be expressed well in the bacterial host. The libraries of the invention are based on human germline derived templates that are well-expressed and purified from standard recombinant mammalian hosts (e.g. HEK 293 and CHO cells) as well as bacterial hosts, and have high stability and good solution properties.

Maturation. The large number of positions in the V-region sequences that can impact recognition of antigen, coupled with potential variation of up to 20 different amino acids at each position preclude the practicality of including all variations in a single library. Human antibodies achieve high affinity and specificity by the progressive process of somatic mutation. The libraries of the invention are designed and ordered to permit parallel selection and targeted variation while maintaining the sequence integrity of each antibody chain such that they reflect human antibodies.

Alternatives design. The above design recapitulates natural human antibodies. The modular nature of the system is amenable to incorporation of any collection of amino acids at any collection of positions.

Library assembly technologies. Preferred *de novo* antibody libraries are of high diversity (> 10¹⁰), amenable to alteration, and easy to assemble and have a low background of undesired sequences. These background sequences include parental template and low-targeted diversity. Coupling the following methods accelerates library assembly and leads to low background. (a) Kunkle-based single-stranded mutagenesis; (b) Palindromic loop with restriction site; (c) Megaprimer

pIX Fab phage display. All prior filamentous *de novo* human antibody libraries utilize pIII or pVIII phage coat proteins for display, with the exception of a single Fv library (Scripps reference). The combination of pIX with the selected Fab templates is a more efficient selection system for recovering antibodies that retain their selected properties upon conversion into mAbs and other related molecules.

Fab display. Unlike scFv, Fabs are natural segments of human antibodies and they better recapitulate their activity when engineered into full antibodies. Efficient filamentous display of Fabs can require properties beyond good expression in the bacterial host. The V-region templates of the current invention were chosen for efficient display by pIX on filamentous phage.

Phagemid display. The Fab molecule is large relative to the phage pIX coat protein and thus may interfere with assembly of recombinant phage particles if linked to all pIX proteins produced in the bacterial cell. One approach to by-pass this interference is to use a pIX phagemid system, such described by (Scripps reference), whereby both wild-type and Fab-linked pIX proteins can be incorporated into the recombinant phage particle. In a preferred application, libraries of the current invention are displayed by pIX in a phagmid system.

Phage coat protein pIX for display. Like pIII, pIX is present at low copy number on the phage and is amenable to affinity selection of displayed Fabs. However, the pIII protein is critically involved in the infection process and proteins displayed on this protein can interfere with the efficiency of infection. Moreover, either the heavy chain Fd or light chain segments can be fused to pIX for display. The libraries of the current invention displayed on the pIX protein are predicted to be efficiently replicated and presented for selection and/or screening.

Fab-pIX expression. One approach to screening Fabs recovered from phage libraries is to remove the phage coat protein that is linked to the Fab molecule for display. The small size of the pIX protein provides the option of production of screening of Fabs directly without this step.

**DESIGN LIBRARY SCAFFOLDS.** The library scaffold is made of a set of human germline VH and VL genes. Analysis literature as well as proprietary antibody information led the identification of the germline genes that represent the human IgG gene families, usage in IgG repertoire and the human antibody canonical structures. Good paring between the Vh and the VL and the probability of successful manufacture of antibody derived from the germline genes were also considered in the analysis. The design of human nature and good paring of the scaffold VH and VL is superior over the design of using a consensus of human germline gene as the library scaffolds (MorphoSys HuCAL GOLD) and is more comprehensively representing human repertoire than designs based on a single germline gene as the library scaffold (Dyax VH and Affytech).

**EXPRESSION AND DISPLAY ABILITY OF THE LIBRARY SCAFFOLDS.** Good expression and display ability of the library scaffold Fabs directly relate to the quality of the library to be developed over the scaffold genes. The library scaffold Fab expression and display ability was examined before the library construction. A few scaffold Fabs that expressed but unable or poorly displayed were excluded from the library construction. The well expressed and displayed library scaffolds ensures that high proportion of the Fabs in the library are functional, more superior than the libraries derived from combinatorial cloning of VH and VL genes genetically amplified from a natural sources (CAT).

**DESIGN H-CDR3 DIVERSITY.** The library VH-CDR3 was diversified in both lengths and sequences. About 10⁹ to 10¹⁸ total sequence possibilities depending on the lengths of the CDR were designed in VH- CDR3 reflecting the importance of VH-CDR3 in antigen binding. Unlike the use of all amino acid codons for amino acid diversification (e.g., NNK by Genentech 2004) and the use of exhaustive cloning methods to engineer VH-CDR3 sequences from natural sources (CAT, Dyax, Affytech), we created VH-CDR3 by using designed oligo-nucleotides that encode amino acid codons mimicking the amino acid usage pattern in human IgG repertoire. The designed VH-CDR3 contains fewer un-wanted (e.g., stop codon) and fewer un-favored amino acid (e.g., cystine) but more proportion of IgG like CDR3 sequences. The degenerated olignucleotides can be readily used to generate a large size gene library in a mutagenesis reaction.

**DESIGN H-CDR1 AND H-CDR2 DIVERSITY.** Amino acid diversity in VH-CDR1 and VH-CDR2 were designed to mimic the variations within the germline sequences. The combined total sequence diversity in VH-CDR1 and VH-CDR2 is ranged between 10²-10⁵. Combining with the large VH-CDR3 sequence variations this increases total sequence variations in the library and hence increases chances of identifying antigen binding antibodies. The unique design of the small, germline like diversity favors of discovering germline or natural like antibody sequences and minimizes the potential of isolating non-natural CDR combinations due to combinatorial effect on the library creation. This design is different from many of other synthetic or semi-synthetic antibody libraries that contained larger sequence variations in all three VH CDRs (Genentech, Dyax and MorphoSys) and yet only small portion of the large theoretic sequence diversity was able to be captured in the library.

**DESIGN DIVERSITY IN LC.** The library light chains were strategically designed to be varied in the de novo library and the Lc diversities were introduced into the CDRs after Fabs being selected via phage panning. As the Lc diversity in the antigen binding Fabs is designed for binding affinity improvement, CDR positions that found frequently in contact with the antigen in antigen-antibody complex of known structure were chosen for diversification. The step-wised CDR diversification strategy effectively manages the large theoretic CDR sequence diversity and generates high binding affinity antibodies, more suitable for biological characterization.

**METHODS FOR LIBRARY GENERATION.** A modified Kunkel mutagenesis method, which efficiently generates billions of E. coli colonies each harboring a different Fab sequence, is used for the generation of the large Fab libraries. While efficient, percent of non-mutagenized parental DNA increases when adapted in generation of a highly sequence complex library. In addition, technical limitation of synthesis long oligonucleotides reduced the effectiveness of the method to making libraries containing sequence diversities in distance regions. To overcome the limitations, additional techniques of generating oligonucleotide >350 bases (mega-primer) and of creation of a stem-loop sequence containing a restriction enzyme recognition site in the mutagenesis template were used in combination of the standard Kunkel mutagenesis method. Comparing to other library technologies, such as restriction cloning (MorphoSys, CAT and Dyax), phage recombination (Giggapack, Invitrogene) and sequence specific recombination (CAT), used by others to for library generation, the improved Kunkel based method is significantly more effective in generation of >10⁹ sequence diverse library and is more versatile to introduce sequence diversity in any location on the targeted DNA.

**IN-LINE AFFINITY MATURATION.** An integrated affinity maturation process, or in-line affinity maturation, is designed to improve binding affinity of all Fabs selected from the library. As the library is strategically designed to leave the VL of the Fab in vary, additional antigen binding sites should be readily created in VL via CDR sequence diversification. Improving binding affinity of all Fabs after panning increase the success of identifying therapeutic antibody lead. The use of Kunkel method for library generation ensures the effective execution of the VL sequence diversification strategy in a simple and continues process. The design strategy and the technically advantages of using the improved Kunkel mutagenesis method made the approach superior over other pooled maturation strategies reported by CAT and MorphoSys where tedious library generation methods are employed that reduce the efficiency and benefit of the purpose.

**PARALLEL LIBRARY PANNING.** A parallel panning process using a state-of-the-art, semi-automated equipment is developed to process the individually made sub-libraries. The parallel panning maximizes the potential of discovery a diverse set of antibodies, supporting the uniquely designed and generated libraries. Effective use of parallel panning in in-line affinity maturation also enables antibodies having a wide range of affinity to be improved simultaneously. Development of a machine based panning also allows systematic examine different panning conditions to discovery antibody of desired properties.

**AFFINITY RANKING.** Affinity based binding assays are applied to the large, diverse and high affinity antigen specific binding antibodies to select the best binding antibodies for further characterization. Standard biochemical methods like ELSIA as well as affinity measuring equipments, for example, BIAcore, Octet and BIND that are suitable for processing large number of samples are used alone or in combination for this purpose.

While having described the invention in general terms, the embodiments of the invention will be further disclosed in the following examples that should not be construed as limiting the scope of the claims.

### EXAMPLE 1: Example 1. Design library scaffolds

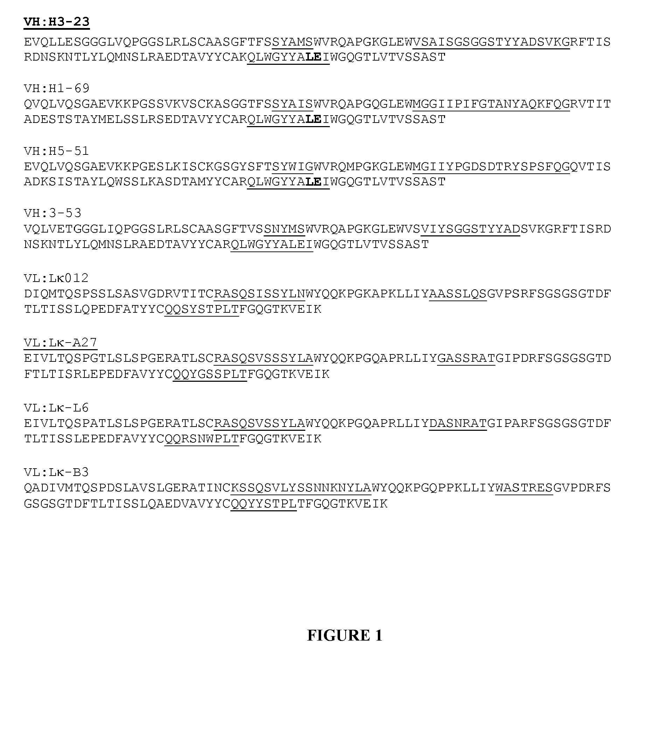

Human germline genes were designed as the library backbones. A panel of germline VH and VL genes were initially identified based on the properties of 1) usage in naturally expressed IgG, 2) structure (main chain conformation) topology favoring for protein and peptide antigen recognition, 3) biochemical and biophysical properties of antibodies derived from the germline genes and 4) likelihood of forming of VH and VL hetero-dimer as an antibody. Four (4) VH and four (4) VL germline genes from the panel are further selected that are best representing the properties listed above. A single, artificial H-CDR3 sequence of 10 amino acids derived from a known antibody with sequence modifications and the human JH4 segment were used in combination with each germline VH to complete the complete VH sequences. For the VL, Jκ1 segment was used to combine with each of the selected germline VL. These 4 VH and 4 VL germline based genes are chemically synthesized. Combination with a defined CH and a CL sequence, they constituted sixteen (16) recombinant human Fabs. For VL-λ, the Jλ2 segment was combined with each of the selected VL-λ germline scaffolds. Three VH (169, 323 and 551) germline genes were combined with the VL-λ germline scaffolds and cloned into vectors containing the CH1 and Cλ sequences to yield 12 recombinant human Fabs. These 28 Fabs were used as the library scaffold or template to display sequence diversity in heavy and/or Lc chain complimentarily determining regions (CDRs). Figure 1 shows the sequences of the designed four VHs and four VLs.

### Example 2. Expression and display of the library scaffolds

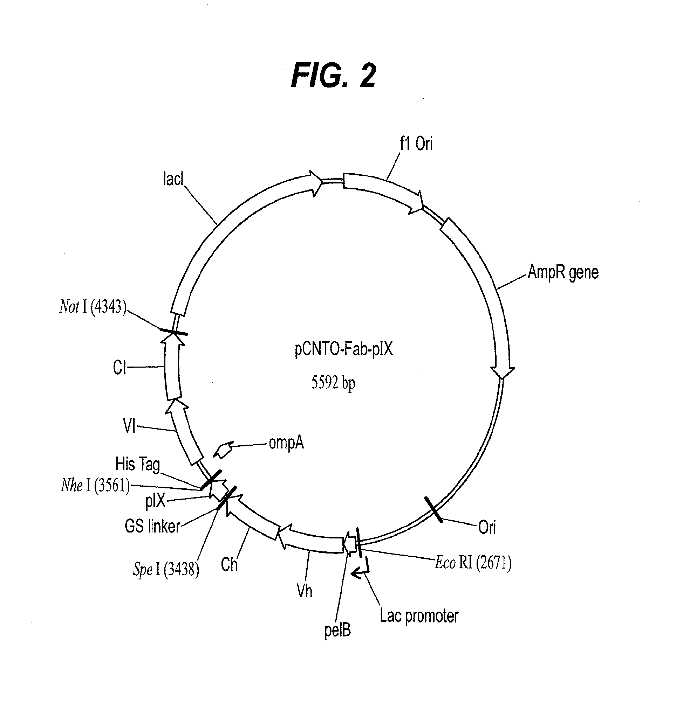

The synthetic Fab DNA are cloned into the pCNTO-lacI-pIX vector (Fig --). The VH gene was cloned via the Ncol and Apal sites. The VL gene, including the ompA signal sequence and sequences upstream was cloned via Nhel and BsiWI sites (Figure 2). The expression and display of the scaffold Fabs were examined in a Western Blot analysis and in a phage ELISA, respectively.

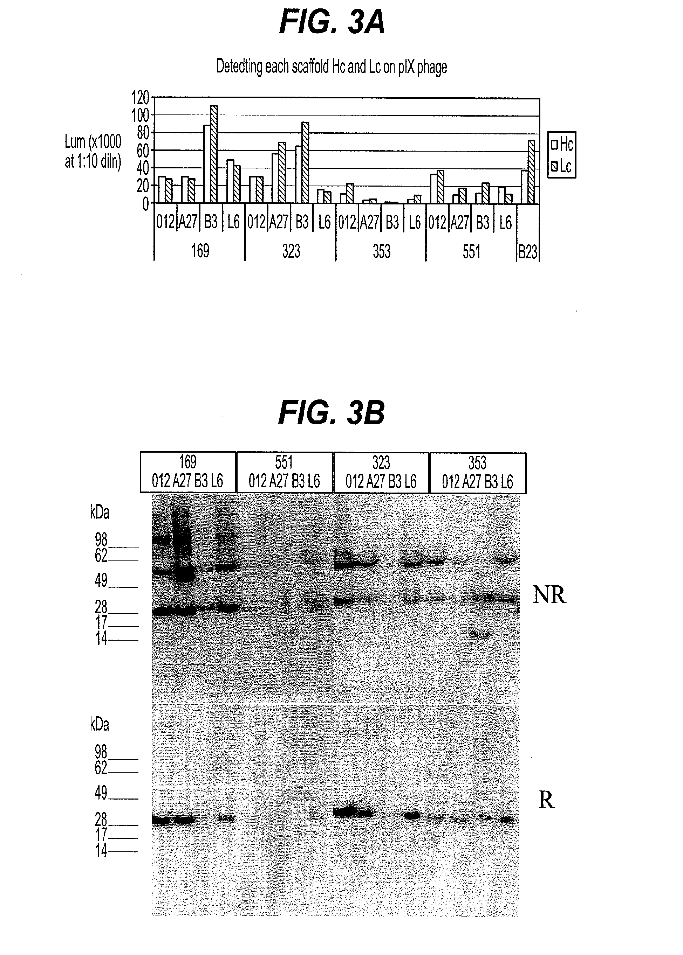

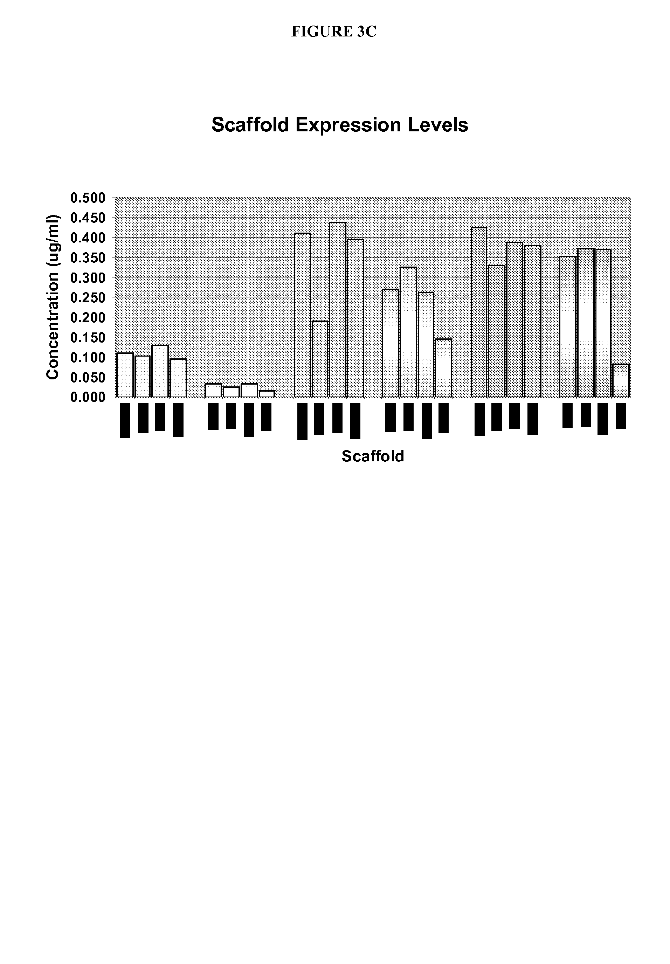

To examine display, phage was prepared and tested in a phage ELISA. Briefly, pCNTO-Fab-pIX-lacI construct was transformed into the MC1061 F' cells and grown over night in 2xYT / Carb (100ug/ml) / TET (15ug/ml) / 1% Glucose medium at 37oC with shaking. Next morning 50ul of this growth was used to seed 5ml of 2xYT / Carb (100ug/ml) and grown to an OD600 of 0.6-0.8. The culture was then infected with (# of phage) of helper phage for 40 minutes at 37oC without shaking. The infected cells were spun down, resuspended in 5ml of 2xYT / Carb (100ug/ml) / TET (15ug/ml) / Kan (35ug/ml) / 0.5mM IPTG medium, and grown over night at 30oC with shaking. The following morning cells were spun down and phage supernatant was collected and used for phage ELISA. 50ul of phage supernatant (neat) as well as 3 serial dilutions of 1:5 were added to ELISA wells coated with anti-Fd(Hc specific) or anti-kappa (Lc specific) antibodies. After incubation, the unbound phage was washed away and the binding phage was detected by anti-M13 antibody. All scaffold combinations except H3-53/L-B3 and H3-53/L-A27 showed good binding to anti-Fd and anti-kappa antibodies, indicating sufficient display of both Hc and Lc on the phage surface (Figure 3).

To examine Fab expression, the pIX gene is first excised from the pCNTO-Fab-lacI-pIX vector by Spel and Nhel restriction enzyme digestions followed by self-ligation of the digested vector DNA. This creates the Fab expression construct, pCNTO-Fab-lacI (Figure 2). M1061F' cells harboring the pCNTO-Fab-lacI phagemid were grown overnight in 2 X YT/1%glucose/Amp (100ug/ml) medium at 37°C with shaking. An aliquot of 5ml of the overnight medium was used as un-induced control. 0.1ml of the overnight culture was inoculated into 10ml 2XYT/0.1% glucose/Amp (100ug/ml) culture medium. The culture was grown at 37°C with shaking to OD₆₀₀ₙₘ 0.8 - 1.0. IPTG was added to a final concentration of 0.5mM to induce Fab expression. The culture continued to grow for an additional 16-20 hours at 30°C with shaking. The induced cultures were spun down and each cell pellet lysed by adding 0.4mL BPER II reagent (Pierce). The supernatant of the spent cell lysate was collected and analyzed by Western Blot for Fab expression. All Fabs expressed well at (> 2ug/ml), with the exception of those containing the B3 LC which expressed at about a 10 fold lower level. Fab expression was also examined using an ELISA. 50ul of spent cell lysate as well as 6 serial dilutions of 1:5 were added to ELISA wells coated with anti-Fd(Hc specific). Fab was detected with either anti-kappa or anti-lambda HRP conjugated antibodies. Fab amounts were quantitated relative to a Fab control standard curve (Figure 3C).

### Example 3. Design of H-CDR3 diversity

We designed large sequence diversities in H-CDR3 mimicking the natural immune repertoire. A total of about 5250 non-redundant and complete (including v region, CDR3 and framework 4) human antibody sequences were collected from various public sources. The data set contained all 7 Vh families. Proprietary programs were written to extract CDR3 segment based on Kabat definition. The antibody sequences were further analyzed based on CDR3 length. The lengths of H-CDR3 ranged from 1 to 27 amino acids, and were shown as a normal distribution. The largest number of antibodies contained 12 aa in H-CDR3. Antibody sequences with the same length were aligned and grouped. The JH6 derived sequences were subtracted from the length groups to minimize the JH6 associated, multiple and consecutive tyrosine containing sequences. Programs were written to calculate amino acid distribution for each length. Residues at each position of the H-CDR3 length sequence group were sorted based on their frequency. An internally installed Weblogo (9-Crooks GE, Hon G, Chandonia JM, Brenner SE WebLogo: A sequence logo generator, Genome Research, 14:1188-1190, (2004)) was used to plot a distribution for each HCDR3 length. We decided to working on the sequences containing 7-14 amino acids of H-CDR3, which approximately covered about 65% for total human antibody repertoire.

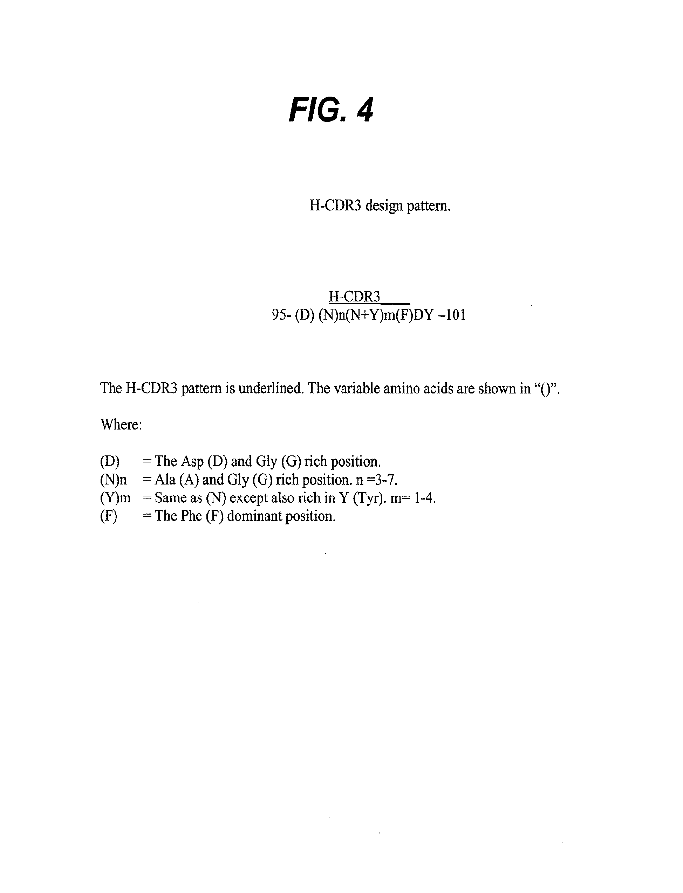

By analyzing the amino acids distribution, we identify a pattern that describes the amino acids distribution of H-CDR3 containing 7-14 amino acids. Figure 4 showed the pattern of H-CDR3. Despite of highly diverse amino acids observed in H-CDR3, amino acids Glycine and Alanine are frequently used in all positions. In addition, Aspartic acid (D) is frequently used in position 95 and Tyrosine (Y) is frequently encoded in the positions closer to the J segment in H-CDR3. Although sequence at position 99-101 varies, amino acids Phenylalanine (F), Aspartic acid (D) and Tyrosine (Y) are predominantly used in IgGs at these positions. Since these positions often serve as structural support to H-CDR3 and are less accessible to antigen and/or to surface of IgG, we fixed amino acids F/L at 99, D at 100 and Y at 101, respectively.

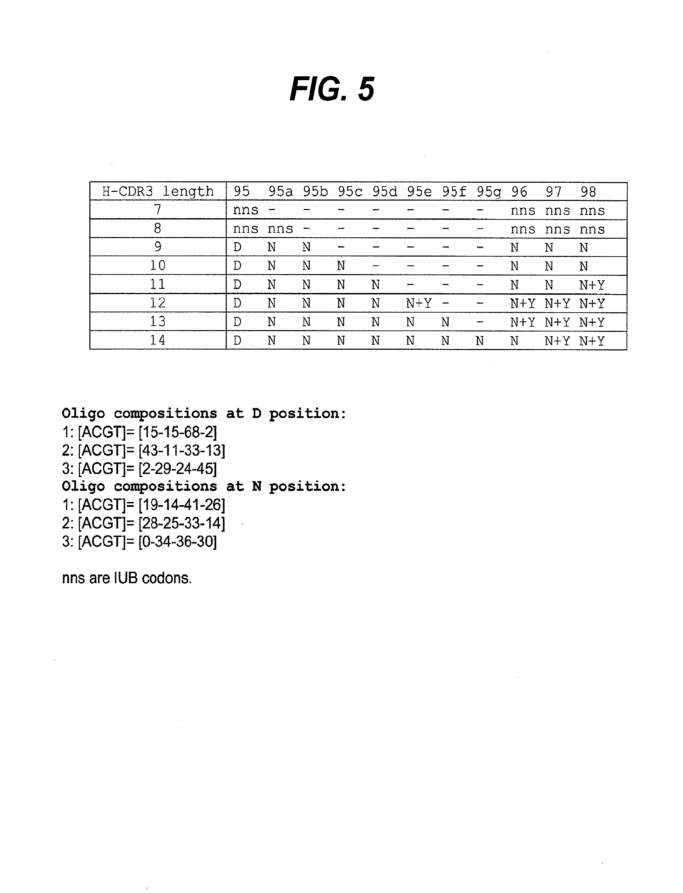

To mimic the highly diverse amino acid distribution in H-CDR3, we designed mixed-base oligonucleotide to encode H-CDR3 sequence and used them in library construction. A procedure described by Wang & Saven (10-Wang W, Saven JG. Designing gene libraries from protein profiles for combinatorial protein experiments. Nucleic Acids Res. Vol 30, No 21, e120. 2002) is used to determine the initial values of nucleotide mixture ratios at each position of a codon triplet in multiple random trials so that the coding amino acids derived from the codon triplets mimic the targeted amino acid distribution. In order to minimize the STOP codons (<3%) and reduced the Cysteine codon by the mix-base oligonucleotides, we further refined values of nucleotide mixture ratios at each position of a codon triplet using a script in Solver of MicroSoft Excel™ against the same target function as the Wang & Saven procedure. The nucleotide composition used for D and N positions are shown in Figure 5. For the shorter H-CDR3 lengths, e.g., 7-8 amino acids, the NNS codon is used instead.

We then generated mixed-base oligonucleotides based on the codon design. In addition to the D and N position, the Tyrosine (∼18% Y) rich positions share otherwise the similar aa distribution as in N positions. Separate oligos with N design codon at N position plus fixed codon TAT at each Tyrosine position were made and used to mix with the N design codon oligos for libraries carrying CDR lengths 11 to 14 amino acids. The ratio used to mix N codon oligo and N plus TAT codon oligos for a particular Y position is about 7:1. Since the designer N codon gives about 4.7% for tyrosine, this mixture results in an additional 13% tyrosine from the fixed TAT codon at Y position.

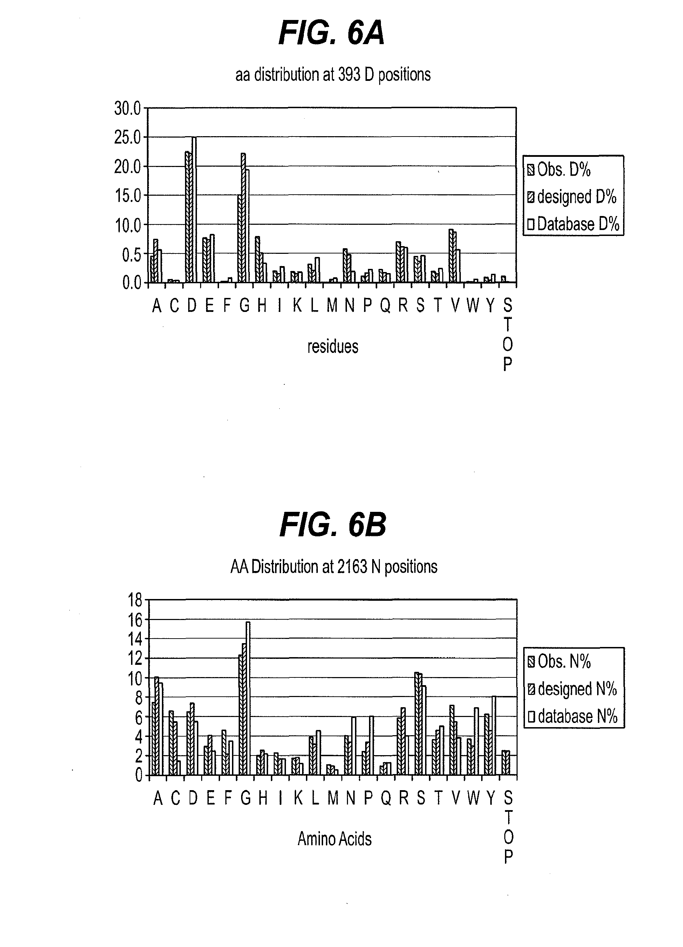

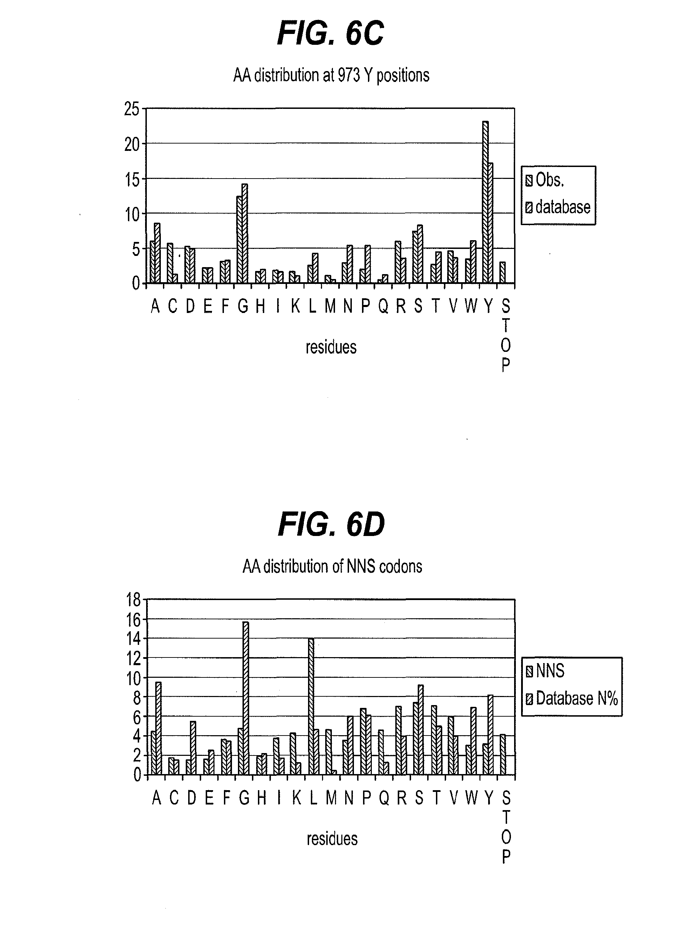

The oligos were used to construct the de novo Fab library using the methods described in example 6. DNA sequences of about 100 colonies from each sub-library were obtained and analyzed. Clones that were mutated from TAA stop codon in H-CDR3 were considered positive clones. The translated H-CDR3 sequences were used to determine amino acid distributions at individual or combined D, N and Y positions. The result showed that the observed amino acid distribution at these positions closely mimicked the distribution found in the database and in the design (Figure 6). Also, the designed mix-base oligo showed better mimicking of the amino acid distribution at these positions to the design and database than does by the NNS degenerated oligos (Figure 6).

### Example 4. Design H-CDR1 and H-CDR2 diversity.

We design the diversities in H-CDR1 and H-CDR2 to mimic the germline gene repertoire. H-CDR1 & CDR2 positions that are targeted for diversification are determined by 1) diversity in germline genes, (11-Vbase) and 2) frequency found in contacting with antigen in antibody-antigen complexes of known structure determined by solvent exposure analysis and by literature (12-Almagro 2004). The amino acids diversity at the positions are determined by 1) usage in germline (11), 2) amino acids that are most frequently used in the IgGs derived from the germline genes (proprietary database), 3) amino acids predicted to be derived as the results of somatic mutations and 4) biochemical and biophysical properties of amino acids that contribute to antigen recognition. The combined sequence diversity in H-CDR1 & CDR2 ranges from 10² to 10⁵. The usage frequency was used as a filter to restrict amino acids that are not frequently used in IgG. This minimizes the non-natural sequence created by combinatorial mutations. Table 1 showed the sequence analysis and the designs of H-CDR1 and CDR2 diversity.

Degenerated oligonucleotides best covered the H-CDR1 and H-CDR2 design are designed and synthesized (Table 1). The oligonucleotides are used as primers in a single Kunkle's ssDNA dependent mutagenesis reaction. The designed degeneracy at the defined H-CDR1 & CDR2 positions are thus introduced into H-CDR1 and H-CDR2 as expected. Sequence analysis randomly picked clones following the Kunkel mutagenesis reaction showed that 31% to 55.7% of the clones sequenced had mutations in H-CDR1 and/or H-CDR2 regions (Table 2). The percentage of clones with both H-CDR1 and H-CDR2 mutations were found to be equal or higher than that of clones have mutations in only one CDR region.

### Example 5. Design diversity in Lc

The following parameters are considered in design light chain CDR diversity. First, we defined that the combined Lc diversity should not exceed 10⁸, as the Lc libraries are used in affinity maturation and thus are combined with a pool of approximately 10² unique Hc clones derived from the phage selection during the first stage of panning the libraries. Therefore, the combined Hc and Lc complexity is about <10¹⁰, which can be effectively captured in a library generated by a single Kunkel's mutagenesis reaction. Second, we targeted for diversification positions frequently found in contact with the antigens in antigen-antibody complexes of known structure or so-called Specificity-Determining Residues (SDRs; 12); that is, positions 30-32, 50 and 91-94, for L-CDR1, L-CDR2 and L-CDR3, respectively. Third, position 96 is also variegated due to it is encoded in the Joining (J) genes and all the five human J genes differ at that position. Fourth, same as the concept used in design H-CDR1 and H-CDR2, we minimized the sequence diversity that not likely occur in nature by choosing the germline encoded amino acids that is also frequently found in the rearranged IgG at the positions to be diversified. Finally, we evaluate the biochemical and biophysical properties of the amino acids suitable for antigen binding at the giving positions (13-Tomlinson et al., 1995) and complemented the V germline gene diversity with those amino acids to reach the 10⁸ criteria. The individually designed Lc-CDR diversities are shown in Table 5.

An overlapping PCR method is used to generate the Lc containing the designed diversities at the specific positions. Degenerate codons can be placed at positions within amplification oligos that span individual CDRs. These degenerate codons encode the designed amino acids at the CDR positions. Alternatively, several oligos encoding the specific desired codons at each position can be synthesized. These oligos can then be mixed to generate a library that will be used to amplify the library DNA used in overlap PCR. The designed oligos are used to amplify fragments of an Lc, generating a pool of sequence variants at the desired SDR positions. Lc fragments encoding the framework #1 region, CDR1-2 mutated region and the Framework #3-CDR3 mutated region were synthesized by PCR with degenerate oligos. The before mentioned VL library fragments were then combined in an overlap-PCR with flanking amplification primers. This reaction produces a full length VL library with sequence variations in all three CDRs at the desired positions.

Alternatively, the DNA synthesis method, "GeneWriter" can be used to synthesize the individual Lc libraries according to the designs. The nucleotide sequences can be back translated from the designed amino acid sequences using E. coli preferred codon. Oligonucleotides, each covers a part of the designed sequence and contains the designed nucleotide variations can be synthesized and assembled as a gene using a technology described in elsewhere (14-US patent 0165946 A1 Briefly, the oligonucleotide sequences and designed sequence variations can be processed through the software "MultiWriter" for oligonucleotide generation. Various number of the library oligonucleotides can be generated for the sense strand to represent all combinations of desired sequence variations based. Antisense strand can be designed and synthesized using the same software. The VL genes can be assembled via a series annealing-ligation of the synthetic smaller oligomers as described as "Gene assembler" in the patent (14). The full length VL DNA were amplified by PCR and the PCR products can be used either as a primer in Kunkel's method or as a source for direct cloning into the designed sites in pCNTO-pIX-lacI vector for library generation. Alternative DNA synthesis and large gene assembly methods may also be used to generate the designed VL genes.

### Example 6. Methods for library generation

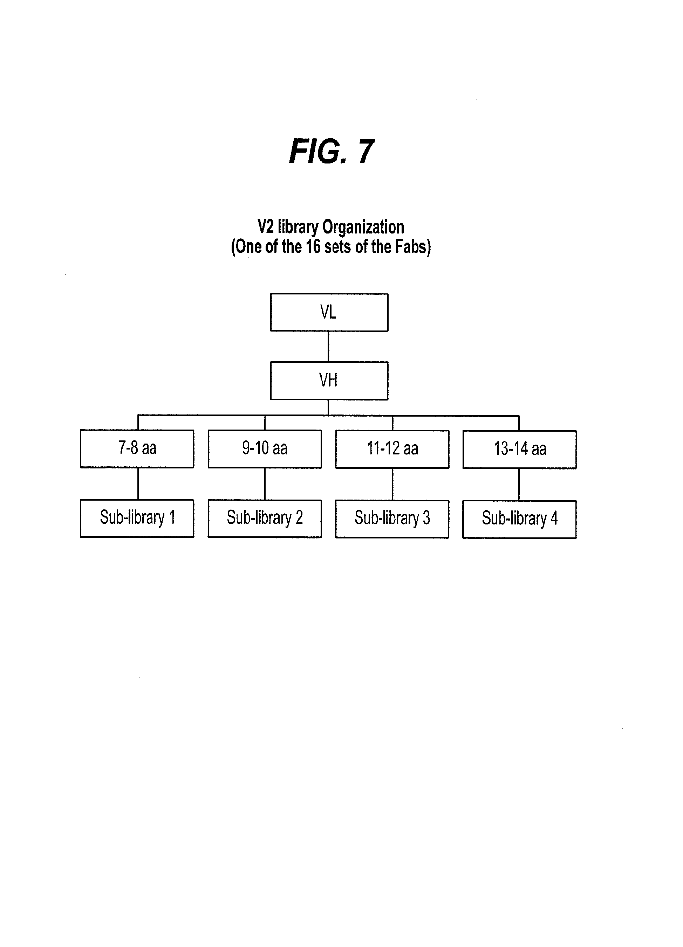

A modified Kunkel's single stranded mutagenesis method is used to make individual library based on each scaffold Fab (15-Genentech paper, 16- Henry Lowman's book). To minimize the effect of the non-mutagenized scaffold Fab template on the final library function, the TAA stop codon was inserted into the H-CDR3 region of the templates. Single strand DNA of each stop codon-containing template was prepared from CJ236 strain. Oligos each encode the designed H-CDR1 and CDR2 diversity and were used simultaneously as primers in a DNA polymerization reaction using T7 DNA polymerase and T4 ligase. The reaction mixture was used to transform MC1 061 F' competent cells, yielding typically > 10⁹ independent colonies per library construction. The colonies were scraped from plates to inoculate fresh media, double strand plasmid DNA was prepared and used to transform CJ236 cells. The transformed cells were infected with helper phage, and single strand DNA was prepared from overnight cell culture and was used as reaction template to introduce H-CDR3 diversity. Library of individual Fab scaffold/template was individually constructed. The 8 different length of H-CDR3 was constructed in four reactions, where H-CDR3 length 7 and 8, 9 and 10, 11 and 12 and 13 and 14 aa were grouped, respectively. Figure 7 shows the library architectures.

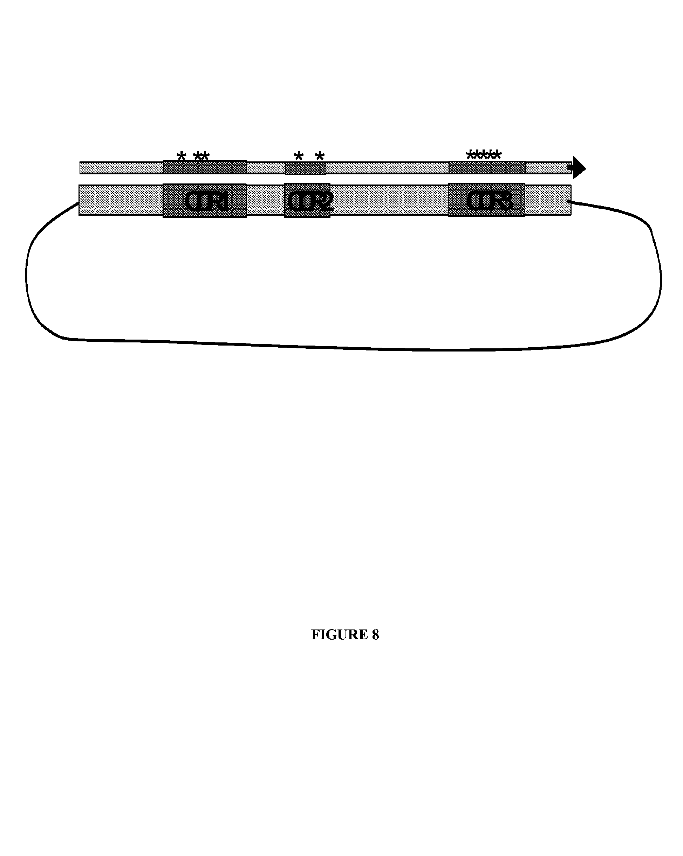

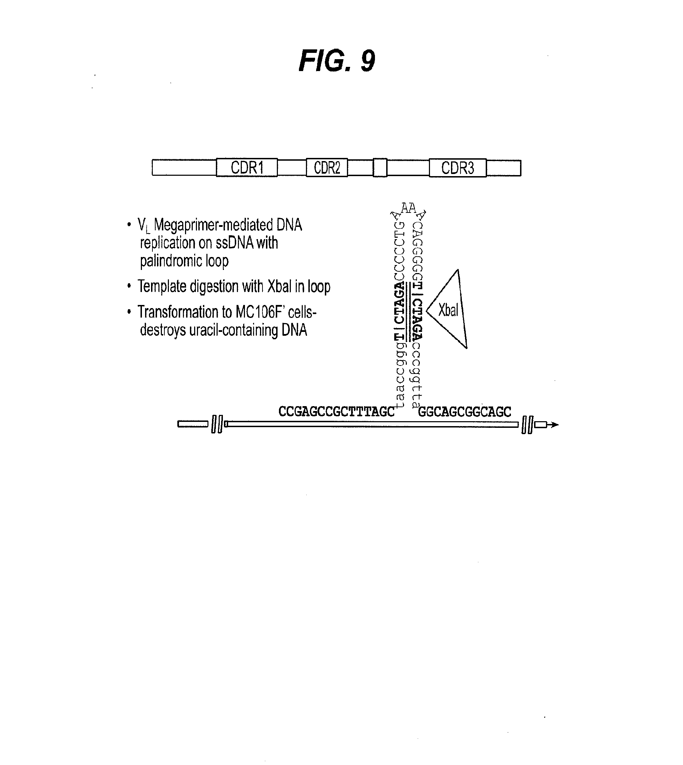

Although efficient, we found the current Kunkel's method often only eliminates un-mutated, parental ssDNA at 20-50% range when constructing a highly sequence complex library. The short oligonucleotide used in the step-wised Kunkel reactions during library generation further reduced efficiency of the method. To further eliminate the parental DNA, a palindrome containing a 6-cutter restriction enzyme site (Xbal) was engineered into the targeted mutagenic sequence regions, e.g., VH and/or VL. During the annealing of primer to ssDNA, the palindrome forms a double stranded step-loop structure and the mutagenic primer spans this palindrome by hybridization to flanking sequences (Figure 8). DNA replication proceeds from the annealed primer using T7 DNA polymerase. Closed circular DNA is generated by T4 DNA ligase. This product is digested with the restriction endonuclease at its site (Xbal) within the palindromic loop, thus nicking the parental DNA strand and destroying its *in vivo* replicative potential. The combined palindrome sequence elimination and the Kunkel's method eliminate the parental DNA greater than 95%, a significant improvement over the current Kunkel method alone. We also generated mega-primers covering all three targeted CDRs to be sequence diversified by overlapping PCR and used in mutagenesis reaction. This allows all combinatorial sequence diversification to be accomplished in one reaction, more efficient than the step-wise reactions using short oligonucleotides. For example, we have generated VL library fragments by overlapping PCR and/or by chemical synthesis using GeneWriter™ and used them as mega-primers for hybridization mutagenesis. The megaprimer is produced by PCR and the minus strand is amplified with a biotinylated primer (Figure 9). The biotinylated strand is captured on streptavidin magnetic beads (Dynal) and the unbiotinylated strand is purified by denaturation in 0.15M NaOH. The eluted strand is then used for megaprimer-mediated DNA replication on ssDNA (REStr420jw).

Libraries with diversity in VH and VL can also be generated by alternative methods and sources. For example, VH and VL genes can be amplified from immunized animal tissues and cloned in-frame with constant region to make Fab libraries (17-winter). Alternatively, the CDR regions from immunized animals, e.g., H-CDR3, can be PCR-amplified and cloned into the correspondent region in the *de novo* library forming a hybrid of immunized H-CDR3 in a *de novo* H-CDR1, H-CDR2 and VL library background. Phage panning of such libraries can isolate high affinity binding antibodies.

### Example 7. In-line affinity maturation

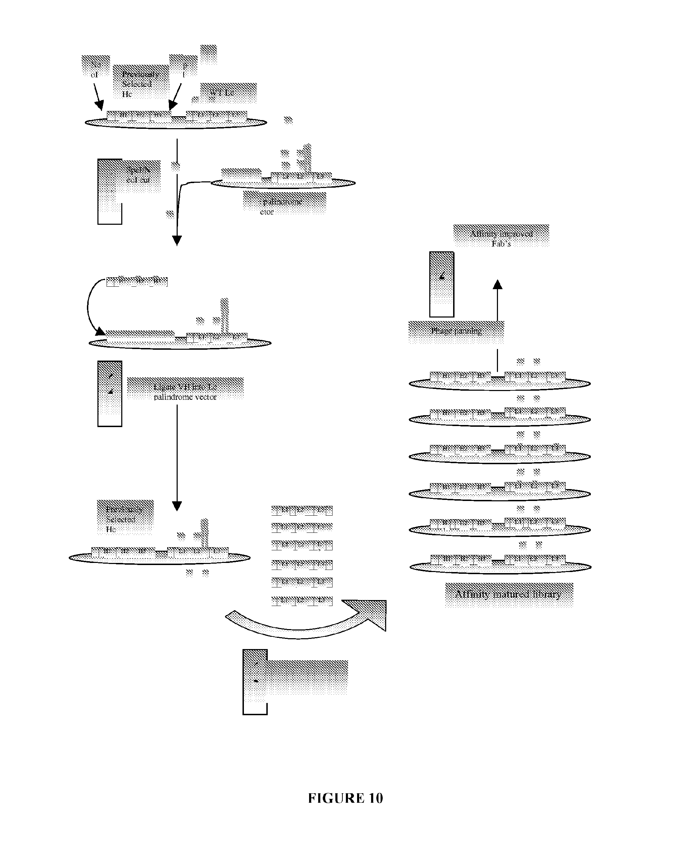

We designed the affinity maturation of the binders selected from the de novo library as part of the phage panning process, and name the process as "in-line maturation". The process starts with a pool of binding antibodies, with or without detailed characterization, followed by the diversification of CDR sequences in Lc of the binders, which were derived from one of the four germline Lc used as the library scaffolds. The Lc CDR diversities will create additional binding activities that can be selected via further phage panning. The processes is described below and also detailed below (Figure 10). First, the VH regions of lead antibodies isolated through panning the Fab de novo library were sub-cloned into VL-Palindromic loop vectors. SsDNA of these vectors are made for hybridization mutagenesis with the VL library megaprimers or suitable methods described in example 6. For example, the combined palindrome sequence elimination and the mega primer Kunkel's mutagenesis method can be used efficiently to generate the VL CDR diversified secondary library for the in-line affinity maturation. Parental DNA is eliminated by Xbal digestion and by subsequent transformation into the non-permissive host strain. This method allowed for highly efficient creation of a library covering designed sequence diversities. Phage library was then made by infection the mutational library with the helper phage. Next the library is subjected to additional rounds of panning with higher selection stringency to enrich binders with improved binding affinity over the binders selected initially from the primary library. These include lowering antigen concentrations, increasing wash time and frequency. Alternatively, binding competitors or other binding stress components, e.g., altering binding temperature and adding detergents, can be included into the binding and/or washing buffer to select binders with desired properties. Other biochemical and biophysical conditions suitable for use in phage binding and subsequent E. coli infection can also be included in the panning. After panning, DNA is recovered from the phage. Fabs are expressed and subjected to binding as well as other biochemical, biophysical and biological characterizations. DNA recovered from the phage can also be cloning directly into IgG vectors. In this case, mAb instead of Fab will be expressed and characterized. For example, we have generated VL library fragments by overlap PCR methods and used them as a megaprimers for hybridization mutagenesis. The mega-primer is produced by PCR and the minus strand is amplified with a biotinylated primer (see example #6). The biotinylated strand is captured on streptavidin magnetic beads (Dynal) and the unbiotinylated strand is purified by denaturation in 0.15M NaOH. The eluted strand is then used for megaprimer-mediated DNA replication on ssDNA. The VH regions of lead antibodies isolated through panning the Fab de novo library were subcloned into VL-Loop vectors. These selected VH/VL-Loop vectors were made into ssDNA for hybridization mutagenesis with the VL library megaprimers. Elimination of parental DNA, as noted by loss of the Xbal-containing palindrome, was 100% determined by sequence of sample of randomly picked library clones. Mutagenesis of the three CDRs to a library of variants was 75%. This method allowed for highly efficient creation of a library of 4 x10⁸ variants. We have seen similar results for the creation of libraries in the other 3 VL-k chains used in the Fab de novo library. Libraries were transformed to 2 x 10⁸ and phage was made to 10¹¹cfu/mL. BSA was panned with the de novo pIX library and hits were isolated (Table 3). Two LC libraries were constructed on primary hits against BSA using the methods described above. The libraries consisted of the F4 HC with A27 LC diversity and a pool of D6, F6, C2, C6 HCs with B3 LC diversity.

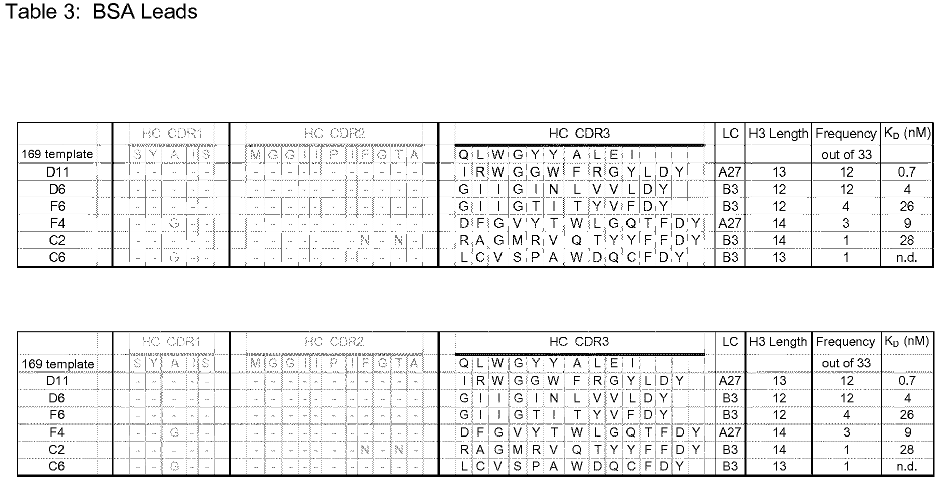

Three rounds of panning were performed with 10nM, 1nM, and 1 nM of antigen respectively using either non-stringent or stringent washing conditions. Sequencing of antigen positive clones was performed and select clones were purified to obtain affinity data. For the B3 library panned with less stringent conditions the majority of enriched clones were wild-type. Of those clones that were not wild-type, none showed enrichment and Biacore data confirmed that they had no improvement in affinity. When the more stringent panning conditions were applied to this same library enrichment of clones with mutations in LC CDR1 were seen and subsequent Biacore analysis showed an improvement in affinity of approximately 3-fold (Table 4). All clones isolated from this library contained the D6 heavy chain, which had the highest affinity for BSA relative to the other heavy chains. For the A27 library, neither panning method yielded clonal enrichment nor recovery of wild-type. While no enrichment of single clones was observed, enrichment in amino acid or amino acid characteristics at particular CDR positions was seen. Select clones demonstrated an affinity improvement up to 10-fold compared to parental (Tables 5 and data not shown).

**Table 4: Stringent panning of B3 library**

| | | | L1 | | | L2 | L3 | | | | | Affinity KD (nM) |
|---|---|---|---|---|---|---|---|---|---|---|---|---|
| | | WT DESIGNED MUTATIONS | Y31 S,Y,H, F,A | K31e K,T,N, E | Y 32 Y,W,D,F,H,S, N,A | W 50 A,D,W,S ,R,Y | Y91 Y,S, H,A | Y92 Y,N,D,S,H,I ,F,K,G | S 93 S,N,T, D,G,H, | T 94 T,Y,L,V,F,A, S | L 96 W,Y, F,L,I, | 1.6 |
| clone # | HC CLONE | Frequency | | | | | | | | | | |
| 27 | D6 | 60 | - | T | W | - | - | - | - | - | - | 0.5 |
| 46 | D6 | 13 | - | - | W | - | - | - | - | - | - | |
| 42 | D6 | 2 | F | - | W | - | - | - | - | - | - | 0.7 |
| 57 | D6 | 1 | - | - | - | - | - | - | - | - | - | |
| 30 | D6 | 1 | - | T | W | - | - | - | - | - | - | |

**Table 5: Non-stringent panning of A27 library**

| | | | L1 | | | | L2 | | | L3 | | | Affinity KD (nM) |
|---|---|---|---|---|---|---|---|---|---|---|---|---|---|
| | | WT | S30 | S31 | S31a | Y32 | G 50 | Y91 | G 92 | S 93 | S94 | L 96 | 1.35 |
| | | DESIGNED MUTATIONS | S,R,N,T, D | N,S,R | S,R,N,A, D,H | Y,F,H,Q, S,E,K | A,D,G, S | Y,S, H,A | Y,N,D,S,H ,I,F,K,G | S,N,T,D, G,H,R | T,Y,L,V, F,A,S | W,Y,F, L,I,R | |
| clone # | HC CLONE | Frequency | | | | | | | | | | | |
| 10 | F4 | 1 | - | - | - | E | S | H | I | H | F | - | 0.18 |
| 13 | F4 | 1 | T | - | - | E | D | H | - | - | Y | - | |
| 22 | F4 | 1 | D | - | R | E | D | H | - | R | F | - | |
| 30 | F4 | 1 | D | - | R | Q | D | - | R | R | Y | - | |
| 34 | F4 | 1 | D | - | - | H | D | - | H | N | F | - | 0.75 |
| 35 | F4 | 1 | T | N | H | - | D | - | Y | - | T | - | |
| 39 | F4 | 1 | - | - | R | E | S | H | - | H | Y | - | |
| 52 | F4 | 1 | R | - | H | Q | A | H | I | H | L | - | |
| 54 | F4 | 1 | - | - | N | K | A | | Y | - | T | - | |
| 70 | F4 | 1 | N | - | A | Q | D | H | - | N | L | - | 0.13 |
| 74 | F4 | 1 | - | - | - | S | D | H | - | H | L | - | |

### Example 8. Parallel library panning

Since our library is designed and constructed in such a way that each sub-library can be used individually based on the used scaffold HC or LC, we designed parallel panning strategy to pan individual as well as sub-pooled library set to maximize chance of identify diverse binding antibodies.

BSA and lysozyme were chosen as model antigens for initial library validation. We pooled 1-69 and 3-23 HC framework sub-libraries into 4 sub-sets by keeping LC framework separate for parallel panning using Kingfisher ( ). As a comparison, we also completely pooled all sub-libraries 1-69 and 3-23 HC and associated LC in one pool and used it to pan against BSA. Biotinylated antigens coated paramagnetic beads (Dyal) were incubated with phage for 1 h at RT. Binding and washings were performed in high throughput format in a 96 well block. After several washing steps, bound phages were eluted and amplified by infecting MC1061F' cells for next round of selection (Example 2 for details). After rounds of selection, DNA was isolated, pIX was removed by restriction digestion and ligated and transformed into MC1061F' to express soluble Fabs. Single clones isolated were tested for Fab expression and antigen binding by ELISA. Fab expression was detected by incubating Fab containing cell extract on anti-Fd coated ELISA plate followed by anti-kappa conjugated HRP. Antigen specificity was tested by capturing biotinylated antigens on streptavidin-coated plates with the subsequent addition of Fab containing cell lysates. For competitive ELISA non-biotinylated antigens in excess amount was included to compete Fab binding. Bound Fab was detected using anti-kappa conjugated HRP.

Results of parallel panning showed 30 of 90-screened clones bound to BSA, representing 33% hit rate in this experiment. The hit rate is much higher than a 7% hit rate when panning was completed using a complete pooled library (Table 6). Although there is no direct comparison of two panning strategies for Lysozyme, where only a completely pooled library panning was performed, the hit rate stands at 4%. (Table 6). Sequence analysis identified 6 unique antibodies for BSA and 3 for Lysozyme are shown in Figure 11.

Unique BSA and Lysozyme binders were purified to determine binding affinity (KD). A two-steps Fab purification procedure (REStm334bw.doc) was used to purify the Fabs. Briefly, E. coli lysate were first subjected a single step IMAC procedure. After dialysis, the IMAC column eluate were subjected a second purification step, anion exchange using QFF Sepharose resin, which improved the purity of the Fab to ∼80-90%. Figure 12 shows the purified BSA and Lysozyme Fabs.

BIAcore analysis was used to determine affinity of the six BSA and three Lysozyme binders. The results showed binding affinities of the Fabs ranged between 0.1 to 28 nM for BSA binders and 6-260 nM for Lysozyme binders, respectively. There is no non-specific binding of either Fab group to BSA, Lysozyme or the reference surface (empty, activated, and blocked CM5 matrix) was detected. Table 7 and 8 show the binding kinetics of the BSA and the Lysozyme binders.

### Example 9. Affinity ranking

To rank the order of the Fabs by binding dissociation constant Koff, crude E. coli supernatants were measured using the Octet system (e.g., as described on public websites, such as forteo.com). The SA sensor probe was coated with test biotinylated antigen. The overnight E. coli induction cultures were lysed with a 2x BBS/lysozyme buffer and placed in 96 assay wells. The calibrated sensor tips were dipped into the assay wells containing the E. coli lysate to measure binding association. The sensor tip was then transferred placed into buffer alone to measure binding dissociation. Binding kinetics was analyzed using the manufacturer's software. For reference, table 6 compares the ranking results derived from Octet and BIAcore with 3 different Fab samples to the same antigen. The binding kinetics were also evaluated using a BIND instrument (e.g., by srubio systems), Fab expressing cell lysates were first filtered to remove un-lysed cells, large cell debris, lipids and sheared nucleic acids. The Streptavidin coated sensor plates were calibrated and incubated with biotinylated antigen. The wells were read for 2 minutes at 30-second intervals and washed 3x with 100 µL/well 1xPBS. Filter treated Fab lysates were then added to the assay wells to allow binding to the antigen displayed on the Streptavidin sensor plate surface. The wells were emptied and 200 µL of 1xPBS was added. The binding signal to the biotinylated antigen was measured from 2 to 5 minutes at30 sec intervals. The Koff rate is obtained by fitting the dissociation data for multiple known amounts of antigen bound to the sensor surface. Although association rates could not be obtained, the dissociation rates, which are independent to Fab concentration, were comparable to Biacore results.

Alternative assay formats to that described above are to display the Fabs by anti-F(ab')2, anti-Fd, or anti-HIS or anti-FLAG as appropriate for the tags included on the Fab protein. The antibody can be conjugated to an amine reactive surface of the sensor plate. Filtered E. coli lysates or periplasmic fractions containing Fab protein are captured by anti-F(ab')2 conjugated on the sensor plate. After incubation with antigen, the binding kinetics can be measure as described above.

Two methods were developed to measure binding dissociation constants (K_{off}) using crude E. coli supernatants. One is an ELISA based method using crude E.coli media supernatant and the other is One is the Octet system (Fortebio website), which is a bench top instrument capable of measuring the binding kinetics of multiple samples at a time. For an affinity ranking ELISA the amount of Fab generated by micro-expression (500uL) was quantitated. A Fab expression ELISA shows the amount of Fab protein in the cell lysate fraction and the spent media fraction. Since both fractions have similar amounts of Fab protein and the cell lysate is more laborious to generate, the spent media fraction was chosen for use in the ranking ELISA. Three antigens were used to establish the ELISA ranking method. They are mouse ST2L, human resistin, and mouse MCP-5. Sequence unique Fabs were used to test their ability to bind to their respective antigen. Multiple dilutions of the spent media containing expressed Fab for each sample were applied to each ELISA. Each dilution of Fab was divided into two, one aliquot for binding to the antigen and the other aliquot for measuring the relative amount of Fab protein containing in each diluted sample. The results of the antigen binding and the expression ELISAs were calculated to establish a specific activity ratio for each dilution point. Plotting the specific activity ratio to the corresponding clone provides a global view of all the Fab samples. From this view it can be determined which dilution lies on a linear point of the curve and captures the most Fab candidates for ranking. Fifty-three mST2L samples were tested at 1:2 and 1:4 dilutions.

The 1:4 dilution was used to determine the rank of these Fabs because it appeared to be in the linear sector of the dilution curve. The ranking of eight mMCP-5 Fabs was done using a broader dilution range, 1:2 and 1:10. The dilution range used here was outside the linear range of the binding curve. Eighty resistin Fabs were diluted at 1:2, 1:4, and 1:8, to determine if three dilutions would better establish a linear range. Only a subset of these Fabs had a linear activity:concentration behavior in this dilution range. The resistin Fabs were expressed again and the spent supernatants were diluted 1:5, 1:10, and 1:20. The 1:5 and 1:10 dilutions did not have samples within the linear titration curve but the 1:20 was within linear range. Specific activity ratios were calculated for all samples and a rank order was established (Tables 9A, B & C). The affinity measurements on Biacore showed very little difference between some of the mST2L Fabs as for their binding kinetics whereas the ranking ELISA data did show a little difference (Table 9A). The disagreement in ranking of Fab 28 between the ELISA and Biacore results could be due to the formats that were used for antigen orientation. The ELISA had the antigen in solution whereas the Biacore format had the antigen displayed on the surface. Fab 40 in the Biacore results shows a fast on-rate (kₐ) giving it the worst KD overall. This may be due to an inaccurate protein concentration. The mMCP-5 Fabs show good correlation in ranking between the ELISA and Biacore results. Fabs S10, S14, and S30 were the lowest ranked binders and Fabs P9, P12, P20, and S1 were the best ranked binders in both assays (Table 9B). Even though Fab P1 was a good binder in the ELISA ranking assay it also showed a reduced signal at the 1:10 dilution and it was a mediocre binder in Biacore.

The resistin Fabs show correlation between the ELISA and the Biacore rankings (Table 9C). Clone 551-22 is the best and clone 169-7 is the worst in the ELISA and Biacore rankings. Further more 323-7 and 323-32 are the next best rankings to clone 551-22 in both ELISA and Biacore rankings.

**Table 9A. mST2L Fabs comparison chart with Biacore results**

| **Fab** | **ELISA Ranking** | **SA ratio 1:4 diln** | ***kₐ* x 10⁵ M⁻¹s⁻¹** | ***k_{d}* x 10⁻⁴ s⁻¹** | **K_{D} (nM)** |
|---|---|---|---|---|---|
| **16** | 3 | 41 | 2.41 | 7.2 | 3 |
| **17** | 1 | 233 | 1.67 | 5.9 | 3.5 |
| **26** | 1 | 220 | 1.47 | 6.31 | 4.3 |
| **28** | 4 | 4 | 2.12 | 0.6 | 3.1 |
| **40** | 2 | 94 | 0.57 | 5.27 | 9.2 |
| **45** | 2 | 71 | 1.44 | 3.8 | 2.6 |

**Table 9B. mMCP-5 Fabs comparison chart with Biacore results**

| **Fab** | **ELISA Ranking** | **SA ratio 1:10 diln** | ***kₐ* x 10⁵ M⁻¹s⁻¹** | ***k_{d}* x 10⁻³ s⁻¹** | **K_{D} (nM)** |
|---|---|---|---|---|---|
| **P1** | 2 | 111 | 7.7 | 2.009 | 2.6 |
| **P9** | 1 | 136 | 20.7 | 0.42 | 0.2 |
| **P12** | 2 | 105 | 74.7 | 2.81 | 0.38 |
| **P20** | 1 | 151 | 11.28 | 0.62 | 0.55 |
| **S1** | 3 | 91 | 28.2 | 1.71 | 0.6 |
| | | | | | |
| **S10** | 6 | 10 | 25 | 15.0 | 0.6 |
| **S14** | 5 | 30 | 4.8 | 4 | 8 |
| **S17** | nd | nd | 30.1 | 7.5 | 2.5 |
| **S24** | nd | nd | 12.6 | 1.88 | 1.5 |
| **S30** | 4 | 49 | 93 | 13 | 1.4 |

**Table 9C. Resistin Fabs comparison chart with Biacore results**

| **Fab sample** | **ELISA Ranking** | **SA ratio 1:8 diln** | ***kₐ* x 10⁵ M⁻¹s⁻¹** | ***k_{d}* x 10⁻³ s⁻¹** | **_{D} (nM)** |
|---|---|---|---|---|---|
| **3** | 4 | 8 | 8.06 | .07 | .5 |
| **7** | 5 | 5 | 1.39 | .41 | .8 |
| | No | | Poor binding | | |
| 2 | binding | o binding | | | |
| 5 | 2 | 13 | 11.4 | .6 | .5 |
| 8 | 2 | 11 | 5.4 | .4 | .7 |
| 8 | 3 | 4 | 12.8 | .99 | .8 |
| 6 | 1 | 25 | 34.8 | .6 | .2 |
| 5 | 3 | 5 | 14.8 | .33 | .6 |

The The ELISA ranking data correlated best to the dissociation values estimated from Biacore interaction analysis except for a few exceptions. The optimized ELISA-based ranking method supports the proper screening and management of large numbers of Fab candidates. Using the Octet instrument, two formats were developed for ranking Fab candidates by kinetic binding activity. The solution format is performed with the Fab applied to the tip sensor surface and the Ag is present in solution. The association rates are calculated when the Ag is binding to the Fab through measurement of bio-mass added to the sensor surface during a period of time. The dissociation rates are calculated when the sensor is placed in a buffer (i.e. 1xPBS) after the Ag is bound to the Fab, resulting in a decrease in biomass on the sensor over time. In the immobilized kinetic binding assay, the antigen is applied to the tip sensor surface and the Fab is present solution. The association and dissociation rates are determined by the Fab increasing and then decreasing the biomass on the tip over time.

The basic protocol for amine reactive sensor tips first starts with establishing an initial baseline followed by the activation step where the first molecule (i.e secondary rgt, Fab, or Ag) is applied to the tip sensor. A quenching step is performed after the first molecule has been bound to the tip sensor and another baseline is established before allowing a binding interaction to occur with another molecule. Either a loading (i.e. adding a Fab or Ag to a secondary reagent coated tip sensor) or association step (i.e. Fab or Ag coated sensor) is performed next. If this is a loading step, then another baseline is established before an association step is done. Once an association step is complete a dissociation step is performed and the experiment is complete. The solution kinetic binding assay uses high-binding SA-coated sensor tips. A sample 96-well plate (cat# 675076, Greiner Bio-One) is prepared as follows: column 1 is filled with 100ul of 1xPBS to baseline the tip sensors, column 2 is filled with Bt-anti-human F(Ab)'₂ (cat# 109-066-097, Jackson Immunological) at a concentration of 10ug/ml in 1xPBS to load the SA coated sensor tip surface, column 3 is filled with 100ul of 1xPBS for the second baseline, column 4 is filled with 100ul of 10ug/ml of Fab samples in 1xPBS for the second loading step, column 5 is filled with 100ug/ml 1xPBS for the third baseline, column 6 is filled with 10ug/ml of Ag in 1xPBS for the association step, and column 6 wells contains 1xPBS for the dissociation step. The kinetics protocol is set up for each step to run at 30°C and shaken at 1000rpm. The first baseline is performed for 100 seconds. The first loading step (anti-human F(Ab)'₂) runs for 600 seconds. The second baseline runs for 200 seconds. The second loading step (Fab candidates) runs for 600 or 900 seconds. The third baseline runs for no more than 300 seconds. The association step (Ag) runs for 600 seconds and the dissociation step runs for up to 900 seconds. The immobilized kinetic binding assay uses amine-reactive sensor tips. The tip sensor box is first incubated in the appropriate MES pH solution (Amine Coupling Kit, Fortebio) at RT for 10 minutes. A sample 96-well plate (cat# 675076, Greiner Bio-One) is prepared as follows: column 1 is filled with the appropriate MES pH solution to baseline the tip sensors, column 2 is filled with a 1:50 ratio of EDC/NHS (amine coupling kit, Fortebio) in the baseline MES pH solutionto activate the sensor surface, column 3 is filled with 10ug/ml Ag in the MES pH solution to load the sensor tip, column 4 is filled with ethanolamine solution (amine coupling kit, Fortebio) to quench un-occupied surface of the Ag loaded sensor, column 5 is filled with 100ul of 1xPBS for the second baseline, column 6 is filled with 10ug/ml of Fab sample in 1xPBS for the association step, column 7 wells is filled with 1xPBS for the dissociation step. The kinetics protocol is set up for each step to run at 30°C and shaken at 1000rpm. The first baseline runs for 100 seconds. The activation step runs for up to 900 seconds. The Ag loading step runs for 600 seconds. The quenching step runs for up to 300 seconds. The second baseline runs for up to 300 seconds. The association step (Ag) runs for 600 seconds and the dissociation step runs for up to 900 seconds. The solution kinetic assay was performed against antigens of 3 different sizes. This extra variable (size of antigen) was used to gain an understanding on how the different bio-mass build-ups would affect the final calculated results for Fab candidate ranking. We found that the larger the size of the antigen yielded better the signal (y-axis) and R² value (Table 13A-C). Amine coupling the Fabs directly to the sensor might help increase the association signal in the solution kinetic assay due to elimination of the 1009kDa molecule, anti-F(Ab)'₂, from the sensor surface, therefore increasing the ability to see the effect of adding the mass of last molecule. The Octet can show differences between Fabs according to their association and dissociation signal curves and they correlate with the calculated rate constants. The mST2L Fabs 16, 26, and 28 have better binding rates than Fabs 1 and 38 (Figure13a). The resistin Fabs 323-11 and 551-7 are the best and Fabs 169-1 and 551-38 are the worst binders (Figure 13b). With the mMCP-5 Fabs it cannot be distinguished in this format the ranking for Ag binding, except that Fab 14 is the worst (Figure 13c). As previously discussed, this format for measuring mMCP-5 may not be the best. The size of mMCP-5 is considerably smaller than a Fab, 9kDa vs 50kDa, plus the tip being coated with a 100kDa secondary reagent will make it even worse for being able to measure a signal difference.

The immobilized assay format provides another means to assess Fab binding kinetics. When the Ag is smaller, e.g mMCP-5, than the Fab as seen in figure 13C of the solution formatted assay, the signal is diminished. Trying to read a small protein on top of a large mass already established on the sensor, has a small effect. Turning the binding partners around where the smaller molecule, mMCP-5, is on the sensor and then reading the binding activity of the larger molecule, e.g. a Fab, should produce a more significant signal. This effect is observed in figure 14, where mMCP-5 is amine-coupled to the sensor surface and association is measured when the Fab is added. The signal increase in this assay format is at least two times greater than that seen in the solution format. Additionally, the association R² values are now greater than 0.85, whereas they were averaging at 0.57 in the solution format. (Table 14)

Biacore is currently the standard for determining binding kinetics. We compared the data from the Octet for the aforementioned Fabs to data from Biacore. There appears to be a difference in sensitivity between Biacore and Octet (Figure 15). The affinity constant, KD, for Biacore is generally higher than the Octet affinity constant. In the Octet experiments the analyte was used at a concentration of 10ug/ml and 1ug/ml with Biacore. Using ten times the analyte for the Octet may cause the association rate constant to appear faster. Also, the dissociation rate constant can be affected indirectly due to excess analyte competing in the assay. In combination this causes the affinity constants appear worse for Octet compared to Biacore. Although not directly proven, this sensitivity issue may have an effect on ranking as well. As for ranking, the resistin Fabs did show some correlation between Biacore and Octet. The only difference with between resistin and the other Ag examples was the difference in molecule size between the Fab and the Ag. Fabs are 50kDa and resistin is 66kDa making them closer in size whereas mST2L at 100kDa and mMCP-5 at 9kDa are different size.

Binding kinetics also were measured using BIND (www.srubiosystems.com), another bench-top instrument, that can measure 96 samples at a time. Fab-expressing cell lysates were first filtered to remove un-lysed cells, large cell debris, lipids and sheared nucleic acids. The streptavidin-coated sensor plates were calibrated and incubated with biotinylated antigen. The wells were read for 2 minutes at 30 seconds intervals and washed 3x with 100 □L/well 1xPBS. Filter-treated Fab lysates were then added to the assay wells to allow binding to the antigen displayed on the streptavidin sensor plate surface. The wells were emptied and 200 □L of 1xPBS was added. The signals for binding to the biotinylated antigen were measured for 2-5 minutes at 30second intervals. A SRU-BIND software subtracts bulk signal obtained from reference wells, where cell lysates without Fab were added. The K_{off} was obtained by fitting the dissociation data to multiple known antigens bound to the sensor surface. Although association rates are very difficult to obtain, the dissociation rates, which are independent of Fab concentration, were comparable to Biacore results.

There are several alternative assay formats to that described above. For example, with the BIND instrument, the Fabs can be captured by immobilized anti-F(ab')2, anti-Fd, or anti-HIS or anti-FLAG on the sensor plate as appropriate. Filtered E.coli lysates or periplasmic fractions containing Fab protein are allowed to be captured by anti-F(ab')2 conjugated on the sensor plate. After incubation with antigen, the binding kinetics can be measure as described above. Also, purifed Fabs can be directly conjugated to an amine reactive surface of the sensor plate and used in this format for measuring antigen binding.

Binding kinetics also were measured using BIND™ (Srubiosystems website), another bench-top instrument, that can measure 96 samples at a time. Fab-expressing cell lysates were first filtered to remove un-lysed cells, large cell debris, lipids and sheared nucleic acids. The streptavidin-coated sensor plates were calibrated and incubated with biotinylated antigen. The wells were read for 2 minutes at 30 seconds intervals and washed 3x with 100 µL/well 1xPBS. Filter-treated Fab lysates were then added to the assay wells to allow binding to the antigen displayed on the streptavidin sensor plate surface. The wells were emptied and 200 µL of 1xPBS was added. The signals for binding to the biotinylated antigen were measured for 2-5 minutes at 30second intervals. A SRU-BIND ™ software subtracts bulk signal obtained from reference wells, where cell lysates without Fab were added. The K_{off} was obtained by fitting the dissociation data to multiple known antigens bound to the sensor surface. Although association rates are very difficult to obtain, the dissociation rates, which are independent of Fab concentration, were comparable to Biacore results.

There are several alternative assay formats to that described above. For example, with the BIND instrument, the Fabs can be captured by immobilized anti-F(ab')2, anti-Fd, or anti-HIS or anti-FLAG on the sensor plate as appropriate. Filtered E.coli lysates or periplasmic fractions containing Fab protein are allowed to be captured by anti-F(ab')2 conjugated on the sensor plate. After incubation with antigen, the binding kinetics can be measure as described above. Also, purifed Fabs can be directly conjugated to an amine reactive surface of the sensor plate and used in this format for measuring antigen binding.

### Advantages

The human Fab *de novo* library described above is distinct from other such libraries because display is via the pIX protein of M13 phage. The use of representative human germline and canonical structure sequences as the library scaffolds and the design of CDR sequences mimicking the natural distribution of amino acids in H-CDR3 make the library comprehensive in coverage of the human immune repertoire. This library design increases the probability of antibody discovery to target antigens in comparison to other *de novo* libraries reported in literature that were built on a single or consensus of germline genes or a mature mAb scaffold. The separate sub-libraries, each composed of a unique scaffold (VH/VL pair) and/or H-CDR3 lengths, coupled with parallel panning of the libraries maximizes the probability for discovery of antibodies of diverse sequence to the target antigen. Further, this provides a mechanism for detailed study of the functionality of each VH/VL pair and their respective sequences and structure in antibody/antigen binding. The integrated in-line affinity maturation process is an efficient method to isolate high affinity antibodies while maintaining diversity.

### References:

1. Kretzschmar & von Ruden, Current Opin Biotechnol. 13:598-602, 2002)
2. Crooks GE, Hon G, Chandonia JM, Brenner SE WebLogo: A sequence logo generator, Genome Research, 14:1188-1190, (2004)
3. Wang W, Saven JG. Designing gene libraries from protein profiles for combinatorial protein experiments. Nucleic Acids Res. Vol 30, No 21, e120. 2002
4. Hennie R Hoogenboom. Nature Biotech. (2005) 23:1105. Selecting and screening antibody libraries.
5. V. Lee, Wei-Ching Liang, Mark S. Dennis, Charles Eigenbrot, Sachdev S. Sidhu and Germaine Fuh JMB (2004) 340:1073-1093. High-affinity Human Antibodies from Phage-displayed Synthetic Fab Libraries with a Single Framework Scaffold.
6. Michela Silacci, Simon Brack, Giulia Schirru, Jessica Mårlind, Anna Ettorre, Adrian Merlo, Francesca Viti and Dario Neri. Proteomics (2005) 5: 2340-2350. Design, construction, and characterization of a large synthetic human antibody phage display library
7. Eskil Soderlind and Carl A.K. Borrebaek. Nature Biotech. (2000). 18:852. Recombining germline-derived CDR sequences for creating diverse single-framework antibody library.
8. Achim Knappik, Liming Ge, Annemarie Honegger, Peter Pack, Melanie Fischer, Gunter Wellnhofer, Adolf Hoess, Joachim Wolle, Andreas Pluckthun and Bernhard Virnekas JMB (2000) 296:57-86. Fully Synthetic Human Combinatorial Antibody Libraries (HuCAL) Based on Modular Consensus Frameworks and CDRs Randomized with Trinucleotides.
9. Griffiths AD, Williams SC, Hartley O, Tomlinson IM, Waterhouse P, Crosby WL, Kontermann RE, Jones PT, Low NM, Allison TJ, Prospero TD, Hoogenboom HR, Missim A, Cox JPL, Harrison JL, Zaccolo, Gherardi E and Winter G. EMBO J 1994: 13(14): 3245-3260. Isolation of high affinity human antibodies directly from large synthetic repertoires.
10. Hans J. de Haard, Nicole van Neer, Anneke Reurs, Simon E. Hufton, Rob C. Roovers, Paula Henderikx, Adriaan P. de Bruýne, Jan-Willem Arends, and Hennie R. Hoogenboom JBC (1999). 274:18218-18230. A Large Non-immunized Human Fab Fragment Phage Library That Permits Rapid Isolation and Kinetic Analysis of High Affinity Antibodies*
11. Rene Michael Hoet and Robert ladner, et al., Nature Biotech. (2005) 23:344. Generation of high affinity human antibodies by combining donor-derived and syntheric complementarity-determining region diversity.
12. Crooks GE, Hon G, Chandonia JM, Brenner SE WebLogo: A sequence logo generator, Genome Research, 14:1188-1190, (2004)
13. Wang W, Saven JG. Designing gene libraries from protein profiles for combinatorial protein experiments. Nucleic Acids Res. Vol 30, No 21, e120. 2002
14. Vbase: The database of human antibody genes. Public website:vbase.mrc-cpe.cam.ac.uk
15. Juan Carlos Almagro. J. Mol. Recognit. (2004) 17:132-143. Identification of differences in the specificity-determining residues of antibodies that recognize antigens of different size: implications for the rational design of antibody repertoires.
16. Ian M.Tomlinson, Jonathan RL.Cox, Ermanno Gherardi, Arthur M.Lesk and Cyrus Chothia. The EMBO Journal (1995) 14:4628-4638. The structural repertoire of the human VK domain.
17. US patent 0165946 A1.
18. European Patent application 90201671.6.
19. US Pat No. 5,942,609.
20. Dillon et al., BioTechniques 9(3): 298-300(1990);
21. Prodomou et al., Protein Engineering 5(8): 827-829 (1992);
22. Chen et al., JACS 116:8799-8800 (1994);
23. Hayashi et al., BioTechniques 17(2):310-315 (1994).
24. Stemmer, Nature 370:370:389-391 (1994)
25. Stemmer, Proc. Natl. Acad. Sci. USA 91:10747-10751 (1994).
26. Sidhu et al, Methods in Enzymology. (2000) 328: 333-363. Phage display for selection of novel binding peptides.
27. Tim Clackson and Henry B Lowma. 2004. Phage display-a practical approach. Oxford University Press
28. Figini et al, J. Mol. Bio. (1994) 239:68-78. In vitro assembly of repertoires of antibody chains on the surface of phage by renaturation.
29. William F. Dall'Acqua, Melissa M. Damschroder, Jingli Zhang, Robert M. Woods, Lusiana Widjaja, Julie Yu, Herren Wu. Methods (2005) 36:43-60. Antibody humanization by framework shuffling.

### TABLES

**Table 1. Heavy chain CDR 1 and CDR2 design**

| Template 3-23 | | | | | | | | | | | | | | |
|---|---|---|---|---|---|---|---|---|---|---|---|---|---|---|
| Position Prevalence of amino acids in IgGs derived from the 3-23 germline sub-family (%) Design Codon Encoded Coverage (Amino acids from 3-23 germline sub-family are shown in red bold.) | | | | | | | | | | | | | | |
| H1-31 | **S** | **D** | N | T | R | G,K,A,V,I,H, M,F,Y,Q,C,L,W,P**SD** | | | | | RRC | **SDNG** | 89 | |
| | 68 | 11 | 9 | 6 | 2 | <1 | | | | | | | | |
| H1-33 | **A** | **G** | **W** | **S** | **Y** | **T** | V,E,N,F,C,P,Q,D,R | | | **AGW** | KSG | **AGWS** | 93 | |
| | 42 | 23 | 22 | 6 | 4 | 1 | <1 | | | | | | | |
| H1-35 | **H** | **S** | **N** | T | Y | R,Q,C,D,G,I,F | | | | **SH** | MRC | **SHNR** | 95 | |
| | 44 | 40 | 9 | 5 | 1 | <1 | | | | | | | | |
| H2-50 | **V** | **A** | **N** | **G** | **Y** | **R** | **S** | **L** | FI, | T **VANG** | *GBG* | **VAG** | 56 | |
| | 27 | 21 | 16 | 8 | 7 | 5 | 5 | 3 | 2 | 2 | | | | |
| H2-52 | **S** | **K** | **N** | **W** | R | T | Y | D,G,I,A | | **SKN** | *ARK*/*TGG* **SKNRW** 96 | | | |
| | 64 | 14 | 8 | 8 | 2 | 1 | 1 | <1 | | | | | | |
| H2-53 | **Y** | **G** | **S** | **Q** | **W** | F | E | H N,P,D,T,A **YGS** *RGC*/*TAT* **GSY** | | | | | 70 | |
| | 29 | 26 | 15 | 12 | 6 | 2 | 2 | 2 | | 1 | | | | |
| H2-54 | **D** | **S** | **N** | G | T | H,E,R,A,B,K,Y,C | | | | **SD** | *RRC* | **SDNG** | 98 | |
| | 56 | 34 | 7 | 2 | 1 | <1 | | | | | | | | |
| H2-56 | **S** | **G** | T | N | D | R,Y,I,A,H,E,V,K,L,F,B,Q | | | | **SG** | *RGC* | **SG** | 85 | |
| | 53 | 32 | 7 | 3 | 1 | <1 | | | | | | | | |
| H2-58 | **T** | **K** | **I** | R | E | A,N,V,M,L,Q,S,G, | | | | **TKI** | *AHA* | **KTI** | 99 | |
| | 99 | 1 | <1 | | | | | | | | | | | |

| Template 5-51 | | | | | | | | | | | | | | |
|---|---|---|---|---|---|---|---|---|---|---|---|---|---|---|
| Position Prevalence of amino acids in IgGs derived from the 3-23 germline sub-family (%) Design Codon Encoded Coverage (Amino acids from 5-51 germline sub-family are shown in red bold.) | | | | | | | | | | | | | | |
| H1-31 | **S** | N | T | D | G | I | R | | | | **SNT** | *AVC* | **SNT** | 94 |
| | 66 | 17 | 11 | 2 | 2 | 1 | 1 | | | | | | | |
| H1-35 | **G** | **S** | A | N | D | T | F | | | | **GS** | *RGT* | **GS** | 84 |
| | 65 | 19 | 10 | 3 | 1 | 1 | 1 | | | | | | | |
| H2-50 | I | **R** | V | M | F | K | S | | L | | **IR** | *ATT* | **I** | 91 |
| | 69 | 21 | 2 | 2 | 2 | 1 | 1 | | 1 | | | *CGT* | **R** | |
| H2-52 | **Y** | **D** | F | G | N | H | | | | | **YD** | *KAT* | **YD** | 91 |
| | 71 | 20 | 4 | 3 | 1 | 1 | | | | | | | | |
| H2-54 | **G** | **S** | Y | D | A | | | | | | **G** | *RGT* | **GS** | 94 |
| | 69 | 25 | 3 | 2 | 1 | | | | | | | | | |
| H2-57 | **D** | **Y** | E | N | S | | | | | | **DY** | *KAT* | **DY** | 91 |
| | 69 | 22 | 5 | 3 | 1 | | | | | | | | | |
| H2-R59 | **R** | **N** | T | K | S | I | | | | | **RN** | *CGT* | **R** | 94 |
| | 72 | 22 | 2 | 2 | 1 | 1 | | | | | | *AAT* | **N** | |

| Template 1-69 | | | | | | | | | | | | | | |
|---|---|---|---|---|---|---|---|---|---|---|---|---|---|---|
| Position Prevalence of amino acids in IgGs derived from the 3-23 germline sub-family (%) Design Codon Encoded Coverage (Amino acids from 1-69 germline sub-family are shown in red bold.) | | | | | | | | | | | | | | |
| H1-33 | **A** | **G** | T | S | V | **Y** | P | F | | **AG** | *GST* | **AG** | 87 | |
| | 57 | 30 | 5 | 3 | 2 | 2 | 1 | <1 | | | | | | |
| H2-50 | **G** | **W** | R | V | L | A | D,T,N,K | | | **GW** | *KGG* | **GW** | 89 | |
| | 66 | 23 | 7 | 1 | 1 | 1 | <1 | | | | | | | |
| H2-52 | **I** | **S** | T | V | M | N | D | K | | **IS** | *ART* | **IS** | 89 | |
| | 68 | 22 | 5 | 2 | 1 | 1 | 1 | <1 | | | | | | |
| H2-53 | **P** | **A** | V | T | G | N,I,S, | D | | | **PA** | *SCG* | **PA** | 93 | |
| | 77 | 16 | 2 | 2 | 1 | <1 | | | | | | | | |
| H2-54 | I | **Y** | M | L | F | V | **TES** | K | | **IY** | *A TC* | **I** | 83 | |
| | 61 | 22 | 4 | 3 | 3 | 3 | 1 | <1 | | | ***TAT*** | **Y** | | |
| H2-55 | **F** | **N** | L | **D** | S,K,Y,A,T,V | | | | | **FN** | *TTT* | **F** | 91 | |
| | 69 | 22 | 5 | 2 | <1 | | | | | | *AAT* | **N** | | |
| H2-57 | **T** | **N** | I | K | D | S | **E** | RP | A | **TN** | *AMT* | **TN** | 87 | |
| | 65 | 20 | 5 | 2 | 2 | 2 | 1 | <1 | | | | | | |
| H2-58 | **A** | **T** | P | G | V | S,R,K,Q,I | | | | **AT** | *RCT* | **AT** | 92 | |
| | 65 | 26 | 4 | 2 | 1 | <1 | | | | | | | | |

| Template 3-53 | | | | | | | | | | | | | | |
|---|---|---|---|---|---|---|---|---|---|---|---|---|---|---|
| Position Prevalence of amino acids in IgGs derived from the 3-23 germline sub-family (%) Design Codon Encoded Coverage (Amino acids from 3-53 germline sub-family are shown in red bold.) | | | | | | | | | | | | | | |
| H1-32 | **N** | **Y** | S | D | K | V | | | | | **NY** | *WAC* | **NY** | 92 |
| | 51 | 39 | 5 | 2 | 2 | 1 | | | | | | | | |
| H1-33 | **Y** | | **A** | N | H | F | C | | | | **YD** | *KA C* | **YD** | 92 |
| | 50 | 42 | 2 | 2 | 1 | 1 | 1 | | | | | | | |
| H2-50 | **V** | **A** | **I** | G | T | L | | | | | **VA** | *GYT* | **VA** | 85 |
| | 48 | 38 | 7 | 3 | 2 | 2 | | | | | | | | |
| H2-52 | **Y** | | **A** | F | H | D | R | | | | **YG** | *TAC*/*GGC* | **YG** | 92 |
| | 53 | 39 | 2 | 2 | 1 | 1 | 1 | | | | | | | |
| H2-S53 | **S** | **T** | **N** | P | R | Y | V | I | G | | **ST** | *TMC* | **ST** | 86 |
| | 50 | 36 | 3 | 3 | 2 | 1 | 1 | 1 | | | 1 | | | |
| H2-54 | **G** | | D | T | V | C | **S** | F | N | E | **GA** | *GST* | **GA** | 82 |
| | 49 | 33 | 6 | 3 | 2 | 2 | 1 | 1 | | | 1 | 1 | | |
| H2-56 | **D** | | T | G | E | R | A | N | | | **DS** | *TCC*/*GAC* | **SD** | 82 |
| | 42 | 40 | 7 | 5 | 2 | 2 | 1 | 1 | | | | | | |

| | | | | | | | | | | | | | | |
|---|---|---|---|---|---|---|---|---|---|---|---|---|---|---|
| **bold: appeared in germline sequences of the same family** ***italic*: nucleotides** | | | | | | | | | | | | | | |

**Table 2. Light chain CDR design.**

| **osition** | **012** | **L6** | **osition** | **A27** | **osition** | **B3** |
|---|---|---|---|---|---|---|
| 30 | SRNAD | RNAD | 0 | SRNTD | | |
| 31 | NSKD | SKD | 1 | NSR | 1 | SYHFA |
| | | | 1a | SRNADH | 1e | KTNE |
| 32 | YWDFHSNA | WDFHSNA | 2 | YFHQSEK | 2 | YWDFHSNA |
| 50 | ADGKYFTN | DGKYFTN | 0 | ADGS | 0 | ADWSRY |
| 91 | YSHA | SGFR | 1 | YSHA | 1 | YSHA |
| 92 | YNDSHIFK | SNHLR | 2 | YNDSHIFK | 2 | YNDSHIFK |
| 93 | SNTDGHR | NTDGHR | 3 | SNTDGHR | 3 | SNTDGHR |
| 94 | TYLVFAS | WA | 94 | TYLVFAS | 94 | TYLVFAS |
| 96 | WYFLIR | WYFLIR | 96 | WYFLIR | 96 | WYFLIR |
| **Diversity** | **1.2 x 10⁷** | **1.5 X 10⁶** | | **2.4 X 10⁷** | | **9 X 10⁶** |

### Lambda

| **Position** | **1e** | **Position** | **1b** | **Position** | **2a2** | **Position** | **3L** |
|---|---|---|---|---|---|---|---|
| 31b | GATND | 31a | NS | 31a | GS | 29 | STNR |
| 32 | DG | 32 | YTA | 31c | ND | | |
| 34 | HN | 34 | SN | 32 | YLR | 33 | SN |
| 50 | GSAD | 50 | DESR | 50 | ED | | |
| 51 | SN | 51 | ND | 53 | NK | 52 | NS |
| 53 | NHY | 52 | ND | | | | |
| | | 53 | KQ | | | | |
| | | 89 | GA | | | | |
| | | 90 | TA | 92 | TA | | |
| 93 | SNRTI | 93 | SD | 93 | SG | 93 | SNTG |
| 94 | SNRTI | | | | SN | 94 | SGT |
| 95a | SHN | 95a | SN | 95a | TN | 95a | ND |
| 95b | GAV | 95b | AG | 95b | FLSPYHVAE | | |
| 95c | SHPL | 95c | DHSGAFNL | | | | |
| 96 | DESIGNE D | 96 | DESIGNED | 96 | DESIGNED | 96 | DESIGNED |
| **Diversity** | 9.20E+06 | | 2.30E+06 | | 1.50E+05 | | 4.00E+04 |

**Table 6. Summary of BSA and Lysozyme panning results**

| | **Total screened** | **Bind to Ag** |
|---|---|---|
| **BSA** | | |
| sub-pool: individual 4 Lc | 90 | 30 (33%) |
| Complete pool | 180 | 12 (7%) |
| | | |

| **Lysozyme** | | |
|---|---|---|
| Complete pool | 180 | 7 (4%) |

**Table 7: Activity of purified Fab and their apparent K_{D} of binding to BSA (40 RU)**

| Fab | *kₐ* x 10⁵ M⁻¹s⁻¹ | *k_{d}* x 10⁻³ s⁻¹ | K_{D} (nM) | Rₘₐₓ (RU) |
|---|---|---|---|---|
| C2 | 0.60 (3) | 1.69 (1) | 28 | 4* |
| C6 | - | - | NB | NB |
| D6 | 5.72 | 1.95 (1) | 4 | 40 |
| D11 | 56.16 (2) | 0.53 (1) | 0.09 | 44 |
| F4 | 0.51 (2) | 0.48 (1) | 9 | 10* |
| F6 | 4.3 (1) | 11.3 (1) | 26 | 43 |

| | | | | |
|---|---|---|---|---|
| NB: no binding | | | | |

**Table 8: Activity of purified Fab and their apparent K_{D} of binding to lysozyme (10 RU)**

| Fab | *kₐ* x 10⁵ M⁻¹s⁻¹ | *k_{d}* x 10⁻³ s⁻¹ | K_{D} (nM) | Rₘₐₓ (RU) |
|---|---|---|---|---|
| E5 | 2.27 (1) | 2.33 (1) | 10 | 51 |
| E7 | 4.6 (1) | 120 (2) | 260 | 50 |
| E8 | 4.24 (2) | 2.49 (1) | 6 | 50.5 |

**Table 13A**

| Tracking ID | **k_{d}** | Error in k_{d} | **kₐ** | **K_{D}** | Assoc R² |
|---|---|---|---|---|---|
| | **[1/s]** | | **[1/Ms]** | **[M]** | |
| 16 | **2.65E-04** | 9.36E-06 | **9.03E+04** | 2.93E-09 | 0.96962 |
| 26 | **1.86E-04** | 1.27E-05 | **1.02E+05** | 1.83E-09 | 0.95989 |
| 28 | **1.86E-04** | 1.65E-05 | **5.08E+04** | 3.67E-09 | 0.98932 |
| 1 | **3.12E-04** | 4.12E-05 | **1.08E+04** | 2.90E-08 | 0.96172 |
| 38 | **2.15E-04** | 2.55E-05 | **1.46E+04** | 1.47E-08 | 0.97907 |

**Table 13B**

| Tracking ID | **k_{d}** | Error in k_{d} | **kₐ** | **K_{D}** | Assoc R² |
|---|---|---|---|---|---|
| | **[1/s]** | | **[1/Ms]** | [M] | |
| 169-3 | **5.69E-03** | 5.24E-04 | **5.37E+05** | 1.06E-08 | 0.61771 |
| 169-7 | **8.47E-04** | 3.47E-05 | **1.93E+05** | 4.39E-09 | 0.77696 |
| 323-11 | **4.47E-04** | 2.37E-05 | **2.99E+05** | 1.50E-09 | 0.79693 |
| 323-32 | **9.89E-04** | 2.69E-05 | **3.07E+05** | 3.22E-09 | 0.80572 |
| 551-7 | **1.16E-03** | 3.23E-05 | **6.97E+05** | 1.67E-09 | 0.69629 |
| 551-22 | **5.39E-03** | 4.48E-04 | **8.79E+05** | 6.13E-09 | 0.66241 |
| 551-38 | **6.29E-03** | 7.70E-04 | **1.59E+05** | 3.97E-08 | 0.45525 |

**Table 13C**

| Tracking ID | **k_{d}** | Error in k_{d} | **kₐ** | **K_{D}** | Assoc R² |
|---|---|---|---|---|---|
| | **[1/s]** | | **[1/Ms]** | **[M]** | |
| P1 | **6.63E-03** | 4.65E-04 | **2.40E+04** | 2.77E-07 | 0.73206 |
| P9 | **3.31E-03** | 1.19E-03 | **2.18E+04** | 1.51E-07 | 0.6118 |
| P12 | **5.76E-03** | 7.73E-04 | **1.93E+04** | 2.98E-07 | 0.57348 |
| P20 | **9.54E-03** | 6.49E-04 | **2.21E+04** | 4.32E-07 | 0.53367 |
| S1 | **5.60E-03** | 6.81E-04 | **2.07E+04** | 2.71E-07 | 0.58443 |
| S14 | **1.01E-02** | 1.16E-03 | **6.03E+03** | 1.68E-06 | 0.43719 |
| S30 | **7.66E-03** | 8.22E-04 | **1.13E+0**4 | 6.77E-07 | 0.52738 |

Table 13A-C shows solution-format kinetic data from Octet. A) Fab, Fab 26, Fab 28, Fab 1, Fab 38 binding to 100kD ST2L was ranked using the Octet by association and dissociation rate constants. B) Fab 169-3, Fab 169-7, Fab 323-11, Fab 323-32, Fab 551-7, Fab 551-22 and Fab 551-38 binding to 66kDa resistin was ranked using the Octet by association and dissociation rate constants. C) Fab P1, Fab P9, Fab P12, Fab P20, Fab S1, Fab S14 and Fab S30 binding to 9kDa MCP-5 was ranked using the Octet by association and dissociation rate constants.

**Table 14:**

| Tracking ID | **k_{d} [1/s]** | Error in k_{d} | **kₐ [1/Ms]** | **K_{D} [M]** | Assoc R² |
|---|---|---|---|---|---|
| P1 | **1.87E-03** | 4.27E-05 | **6.78E+04** | 2.76E-08 | .95439 |
| P9 | **2.69E-03** | 7.78E-05 | **3.88E+04** | 6.93E-08 | .97197 |
| P12 | **6.97E-03** | 1.29E-04 | **1.08E+05** | 6.46E-08 | .85772 |
| P20 | **5.97E-03** | 1.04E-04 | **2.39E+05** | 2.50E-08 | .86825 |
| neg Fab | **6.70E-03** | 2.49E-03 | **-2.38E4*** | -- | .85597 |
| no Fab | **3**.**66E**-**3*** | 2.33E-03 | - | -- | .07006 |

Table 14: Immobilized-format kinetic data from Octet. Fab P1, Fab P9, Fab P12, Fab P20, Fab S1, Fab S14 and Fab S30 binding to 9kDa MCP-5 was ranked using the Octet by association and dissociation rate constants.

**Table 15A: mST2L**

| **Biacore** | **SA display of Bt-Ag** | | | **Octet** | **solution** | |
|---|---|---|---|---|---|---|
| **Fab sample** | ***kₐ* x 10⁵ M⁻¹s^{- 1}** | ***k_{d}* x 10⁻⁴ s⁻¹** | **K_{D} (nM)** | ***kₐ* x 10⁵ M⁻¹s⁻¹** | ***k_{d}* x 10⁻⁴ s⁻¹** | **K_{D} (nM)** |
| **16** | **2.41** | **7.2** | **3** | **0.903** | **2.65** | **2.9** |
| **26** | **1.469** | **6.31** | **4.3** | **1.02** | **1.86** | **1.8** |
| **28** | **2.12** | **0.6** | **0.31** | **0.508** | **1.86** | **3.6** |

**Table 15B Resistin**

| **Biacore** | **Solution** | | | **Octet** | **Solution** | |
|---|---|---|---|---|---|---|
| **Fab sample** | ***kₐ* x 10⁵ M⁻¹s⁻ 1** | ***k_{d}* x 10⁻³ s⁻¹** | **K_{D} (nM)** | ***kₐ* x 10⁵ M⁻¹ s⁻¹** | ***k_{d}* x 10⁻⁴ s⁻¹** | **_{D} (nM)** |
| **169-3** | **8.06** | **2.07** | **2.5** | **5.37** | **56.9** | **0.6** |
| **169-7** | **1.39** | **0.41** | **2.8** | **1.93** | **8.47** | **.3** |
| **323-11** | **11.4** | **0.6** | **0.5** | **2.99** | **4.47** | **.5** |
| **323-32** | **5.4** | **0.4** | **0.7** | **3.07** | **3.89** | **.2** |
| **551-7** | **12.8** | **0.99** | **0.8** | **6.97** | **11.6** | **.6** |
| **551-22** | **34.8** | **0.6** | **0.2** | **8.79** | **53.9** | **.1** |
| **551-38** | **14.8** | **2.33** | **1.6** | **1.59** | **62.9** | **9.7** |

**Table 15C: mMCP-5**

| **Biacore** | **Immobilized** | | | **Octet** | **Immobilized** | |
|---|---|---|---|---|---|---|
| **Fab sample** | ***kₐ* x 10⁵ M^{- 1}s⁻¹** | ***k_{d}*x10⁻³ s⁻¹** | **K_{D} (nM)** | ***kₐ* x 10⁵ M⁻¹s⁻¹** | ***k_{d}*x10⁻³s⁻¹** | **K_{D} (nM)** |
| **P1** | **7.7** | **2.009** | **2.6** | **0.67** | **1.87** | **27.6** |
| **P9** | **20.7** | **0.42** | **0.2** | **0.38** | **2.69** | **69.3** |
| **P12** | **74.7** | **2.81** | **0.38** | **1.08** | **6.79** | **64.6** |
| **P20** | **11.28** | **0.62** | **0.55** | **2.39** | **5.97** | **25** |
| **S1** | **28.2** | **1.71** | **0.6** | **0.39** | **1.63** | **41.3** |
| **S10** | **25** | **15.0** | **0.6** | **1.86** | **3.92** | **21.1** |
| **S14** | **4.8** | **4** | **8** | **6.97** | **19.6** | **28.1** |
| **S30** | **93** | **13** | **1.4** | **3.65** | **16.8** | **46.1** |

Table 15A-C: Comparing kinetic rate constant charts between Biacore and Octet. From top to bottom charts mST2L, Resistin, and mMCP-5 Fab groups.

## Claims

1. An engineered recombinant nucleic acid phage vector for expressing phage display pIX fusion antibody fab fragment amino acid sequences that bind to selected biologically active ligands, comprising
(i) a. a recombinant phage leader coding nucleic acid sequence; operably linked to:
b. a recombinant tag, promoter, or selection coding nucleic acid sequence; operably linked to:
c. a variable heavy chain germline encoding nucleic acid sequence that selectively binds to said biologically active ligand; operably linked to:
d. a portion of a constant heavy chain germline encoding nucleic acid sequence that selectively binds to said biologically active ligand; operably linked to:
e. a peptide linker encoding nucleic acid sequence; operably linked to:
f. a recombinant pIX encoding nucleic acid sequence; and
(ii) a. a variable light chain germline encoding nucleic acid sequence that selectively binds to said biologically active ligand; operably linked to:
b. a portion of a constant light chain germline encoding nucleic acid sequence that selectively binds to said biologically active ligand.

2. An engineered nucleic acid phage vector according to claim 1;
i) wherein said phage leader coding sequence is a pelB sequence,
ii) wherein recombinant tag or selection sequence is a FLAG tag sequence,
iii) wherein said promoter is an inducible promoter,
iv) wherein said inducible promoter is a lac promoter, and/ or
v) wherein said biologically active ligands mediate at least one biological in vivo activity.

3. A bacterial host cell comprising an engineered nucleic acid phage vector according to claim 1.

4. A phage library of bacterial host cells comprising a plurality of engineered nucleic acid phage vectors according to claim 1.

5. A phage library according to claim 4, wherein the biologically active fab antibody fragment variant is expressed as a plurality of fab antibody fragments.

6. A phage library according to claim 5, wherein said plurality is at least one selected from at least 10³, 10⁴, 10⁵, 10⁶, 10⁷, 10⁸, 10⁹, 10¹⁰, 10¹¹, 10¹², or 10¹³ of said variants.

7. A phage library according to claim 5, wherein said variants are generated using a Kunkel mutagenasis method.

8. A phage library according to claim 5, wherein said variants comprise a plurality of diverse heavy chain complementarity determining region (CDR) 3 variants comprised in said fab antibody fragments, wherein optionally said diverse CDR3 variants comprise about 10⁹ to 10¹⁸ sequence variations.

9. A phage library according to claim 5, wherein said variants comprise a plurality of diverse heavy chain complementarity determining region (CDR) 1 and 2 variants comprised in said fab antibody fragments, wherein optionally said diverse CDR1 and 2 variants comprise about 10² to 10⁵ sequence variations.

10. A phage library according to claim 5, wherein said phage library is further comprised of affinity matured fab antibody fragments that have improved binding affinity relative to prior versions of said phage library.

11. A phage library according to claim 10, wherein said fab antibody fragments are panned for increased affinity or biological activity.

12. A phage library according to claim 11, wherein said panning is conducted as part of the library generation process or in parallel using a subset of said phage library.

13. A method for screening a phage fab antibody fragment library for fab antibody fragments having a desired biological activity, comprising (a) expressing fab antibody fragments from a phage library according to claim 9, and (b) selecting bacterial cells expressing said fab antibody fragments having said desired biological activity.

## Patentansprüche

1. Konstruierter rekombinanter Nukleinsäure-Phagenvektor zur Expression von Phagen-Display-pIX-Fusionsantikörper-fab-Fragment-Aminosäuresequenzen, die an ausgewählte biologisch aktive Liganden binden, umfassend
(i) a. eine rekombinante Phagen-Leader-Codiernukleinsäuresequenz; in operativer Verknüpfung mit:
b. einer rekombinanten Tag-, Promotor- oder Selektion-Codiernukleinsäuresequenz; in operativer Verknüpfung mit:
c. einer eine variable schwere Kette keimbahncodierenden Nukleinsäuresequenz, die selektiv an den biologisch aktiven Liganden bindet; in operativer Verknüpfung mit:
d. einer einen Teil einer konstanten schweren Kette keimbahncodierenden Nukleinsäuresequenz, der selektiv an den biologisch aktiven Liganden bindet; in operativer Verknüpfung mit:
e. einer einen Peptid-Linker codierenden Nukleinsäuresequenz; in operativer Verknüpfung mit:
f. einer rekombinanten pIX codierenden Nukleinsäuresequenz; und
(ii) a. eine eine variable leichte Kette keimbahncodierende Nukleinsäuresequenz, die selektiv an den biologisch aktiven Liganden bindet; in operativer Verknüpfung mit:
b. einer einen Teil einer konstanten leichten Kette keimbahncodierenden Nukleinsäuresequenz, der selektiv an den biologisch aktiven Liganden bindet.

2. Konstruierter Nukleinsäure-Phagenvektor nach Anspruch 1;
i) wobei es sich bei der Phagen-Leader-Codiersequenz um eine pelB-Sequenz handelt,
ii) wobei es sich bei der rekombinanten Tag- oder Selektion-Sequenz um eine FLAG-Tag-Sequenz handelt,
iii) wobei es sich bei dem Promotor um einen induzierbaren Promotor handelt,
iv) wobei es sich bei dem induzierbaren Promotor um einen lac-Promotor handelt und/oder
v) wobei die biologisch aktiven Liganden wenigstens eine biologische In-vivo-Aktivität vermitteln.

3. Bakterienwirtszelle, umfassend einen konstruierten Nukleinsäure-Phagenvektor nach Anspruch 1.

4. Phagen-Bibliothek von Bakterienwirtszellen, umfassend mehrere konstruierte Nukleinsäure-Phagenvektoren nach Anspruch 1.

5. Phagen-Bibliothek nach Anspruch 4, wobei die biologisch aktive fab-Antikörperfragmentvariante als eine Vielzahl von fab-Antikörperfragmenten exprimiert wird.

6. Phagen-Bibliothek nach Anspruch 5, wobei es sich bei der Vielzahl um wenigstens eine aus wenigstens 10³, 10⁴, 10⁵, 10⁶, 10⁷, 10⁸, 10⁹, 10¹⁰, 10¹¹, 10¹² oder 10¹³ ausgewählte Zahl von Varianten handelt.

7. Phagen-Bibliothek nach Anspruch 5, wobei die Varianten unter Verwendung eines Kunkel-Mutageneseverfahrens erzeugt werden.

8. Phagen-Bibliothek nach Anspruch 5, wobei die Varianten eine Vielzahl verschiedener Schwerketten-CDR(Complementarity Determining Region)-3-Varianten umfasst in den fab-Antikörperfragmenten umfassen, wobei gegebenenfalls die verschiedenen CDR3-Varianten etwa 10⁹ bis 10¹⁸ Sequenzvariationen umfassen.

9. Phagen-Bibliothek nach Anspruch 5, wobei die Varianten eine Vielzahl verschiedener Schwerketten-CDR(Complementarity Determining Region)-1-und -2-Varianten umfasst in den fab-Antikörperfragmenten umfassen, wobei gegebenenfalls die verschiedenen CDR1- bzw. -2-Varianten etwa 10² bis 10⁵ Sequenzvariationen umfassen.

10. Phagen-Bibliothek nach Anspruch 5, wobei die Phagen-Bibliothek ferner aus affinitätsgereiften fab-Antikörperfragmenten besteht, die eine verbesserte Bindungsaffinität relativ zu früheren Versionen der Phagen-Bibliothek aufweisen.

11. Phagen-Bibliothek nach Anspruch 10, wobei die fab-Antikörperfragmente auf erhöhte Affinität oder biologische Aktivität durchschaut werden.

12. Phagen-Bibliothek nach Anspruch 11, wobei das Durchschauen als Teil des Bibliothekserzeugungsvorgangs oder parallel unter Verwendung einer Teilmenge der Phagen-Bibliothek erfolgt.

13. Verfahren zum Durchsuchen einer fab-Antikörperfragment-Bibliothek nach fab-Antikörperfragmenten mit einer gewünschten biologischen Aktivität, umfassend (a) Expression von fab-Antikörperfragmenten aus einer Phagen-Bibliothek nach Anspruch 9 und (b) Auswählen von die fab-Antikörperfragmente mit der gewünschten biologischen Aktivität exprimierenden Bakterienzellen.

## Revendications

1. Vecteur de phage d'acide nucléique recombinant modifié pour exprimer des séquences d'acides aminés de fragment fab d'anticorps de fusion avec pIX de présentation sur phage qui se lient à des ligands biologiquement actif sélectionnés, comprenant
(i) a. une séquence d'acide nucléique codant pour une séquence de tête de phage recombinante ; fonctionnellement liée à :
b. une séquence d'acide nucléique codant pour une étiquette, un promoteur ou de sélection recombinante ; fonctionnellement liée à :
c. une séquence d'acide nucléique de lignée germinale codant pour une chaîne lourde variable qui se lie sélectivement audit ligand biologiquement actif ; fonctionnellement liée à :
d. une partie d'une séquence d'acide nucléique de lignée germinale codant pour une chaîne lourde constante qui se lie sélectivement audit ligand biologiquement actif ; fonctionnellement liée à :
e. une séquence d'acide nucléique codant pour un lieur peptidique ; fonctionnellement liée à :
f. une séquence d'acide nucléique codant pour pIX recombinante ; et
(ii) a. une partie d'une séquence d'acide nucléique de lignée germinale codant pour une chaîne légère variable qui se lie sélectivement audit ligand biologiquement actif ; fonctionnellement liée à :
b. une partie d'une séquence d'acide nucléique de lignée germinale codant pour une chaîne légère constante qui se lie sélectivement audit ligand biologiquement actif.

2. Vecteur de phage d'acide nucléique modifié selon la revendication 1 ;
i) dans lequel ladite séquence codant pour une séquence de tête de phage est une séquence pelB,
ii) dans lequel la séquence d'étiquette ou de sélection recombinante est une séquence d'étiquette FLAG,
iii) dans lequel ledit promoteur est un promoteur inductible,
iv) dans lequel ledit promoteur inductible est un promoteur lac, et/ou
v) dans lequel lesdits ligands biologiquement actifs médient au moins une activité biologique *in vivo.*

3. Cellule hôte bactérienne comprenant un vecteur de phage d'acide nucléique modifié selon la revendication 1.

4. Banque de phage de cellules hôtes bactériennes comprenant une pluralité de vecteurs de phage d'acide nucléique modifiés selon la revendication 1.

5. Banque de phage selon la revendication 4, dans laquelle le variant de fragment d'anticorps fab biologiquement actif est exprimé sous la forme d'une pluralité de fragments d'anticorps fab.

6. Banque de phage selon la revendication 5, dans laquelle ladite pluralité est au moins l'un choisi parmi au moins 10³, 10⁴, 10⁵, 10⁶, 10⁷, 10⁸, 10⁹, 10¹⁰, 10¹¹, 10¹², ou 10¹³ desdits variants.

7. Banque de phage selon la revendication 5, dans laquelle lesdits variants sont générés en utilisant un procédé de mutagenèse de Kunkel.

8. Banque de phage selon la revendication 5, dans laquelle lesdits variants comprennent une pluralité de variants de région déterminant la complémentarité (CDR) 3 de chaîne lourde divers compris dans lesdits fragments d'anticorps fab, où facultativement, lesdits variants divers de CDR3 comprennent environ 10⁹ à 10¹⁸ variations de séquence.

9. Banque de phage selon la revendication 5, dans laquelle lesdits variants comprennent une pluralité de variants de région déterminant la complémentarité (CDR) 1 et 2 de chaîne lourde divers compris dans lesdits fragments d'anticorps fab, où facultativement, lesdits variants divers de CDR1 et 2 comprennent environ 10² à 10⁵ variations de séquence.

10. Banque de phage selon la revendication 5, où ladite banque de phage est en outre constituée de fragments d'anticorps fab à maturation d'affinité qui ont une affinité de liaison améliorée par rapport à des versions précédentes de ladite banque de phage.

11. Banque de phage selon la revendication 10, dans laquelle lesdits fragments d'anticorps fab sont soumis à adhérence sur plastique pour augmenter l'affinité ou l'activité biologique.

12. Banque de phage selon la revendication 11, où ladite adhérence sur plastique est conduite dans le cadre du processus de génération de banque ou parallèlement en utilisant un sous-ensemble de ladite banque de phage.

13. Procédé de criblage d'une banque de fragments d'anticorps sur phage pour rechercher des fragments d'anticorps fab ayant une activité biologique souhaitée, comprenant (a) l'expression de fragments d'anticorps fab d'une banque de phage selon la revendication 9, et (b) la sélection de cellules bactériennes exprimant lesdits fragments d'anticorps fab ayant ladite activité biologique souhaitée.
